# EUROPEAN PATENT APPLICATION

(11) **EP 3 763 408 A2**
(43) Date of publication of application: **13.01.2021**
(21) Application number: 20180430.9
(22) Date of filing: 17.06.2020
(51) Int. Cl.: A61M 16/00, A61M 16/20

(54) **CPAP SYSTEM AND CPAP APPARATUS**

(30) Priority: 21.06.2019 JP 2019115812; 11.03.2020 JP 2020041917
(71) Applicant: Seiko Instruments Inc., Chiba-shi, Chiba 261-8507 (JP)
(72) Inventor: ENDO, Yoichi, Chiba-shi, Chiba 261-8507 (JP); SUZUKI, Makoto, Chiba-shi, Chiba 261-8507 (JP); KATO, Hisakazu, Hamamatsu-shi, Shizuoka 435-0015 (JP)
(74) Representative: Miller Sturt Kenyon

(57) **Abstract**

The present invention relates to a CPAP system includes a fan that suctions air and delivers the air; a sensor that measures the pressure or flow rate of air; a case having the fan and the sensor built therein and having an air inflow port and an air outflow port t; a motor drive unit that controls a rotation speed of a motor, which rotates the fan on the basis of the pressure or the flow rate, by a PWM signal; a valve that regulates a pressure or flow rate of air supplied from the air outflow port via a tube to a mask worn on a patient; and a valve drive unit that opens and closes the valve on the basis of the PWM signal. The valve drive unit includes a synthesis unit that synthesizes three-phase signals for driving the motor, and a control unit that controls a closing speed of the valve and an opening degree of the valve. The valve drive unit opens and closes the valve on the basis of a synthesized signal obtained by synthesizing the three-phase signals, and the closing speed and the opening degree of the valve controlled by the control unit.

## Description

### Technical Field

The present invention relates to a continuous positive airway pressure (CPAP) system and a CPAP apparatus.

### Background Art

CPAP is a treatment method of sending machine-pressurized air into the airway from the nose to dilate the airway to prevent apnea during sleep. CPAP is an effective treatment method for sleep apnea syndrome.

The CPAP system sends pressurized air into the airway from the nose. The CPAP system includes a CPAP apparatus that delivers air, a tube that sends air at a preset pressure, and a mask that is applied to the nose.

Patients with sleep apnea syndrome wear a mask during sleep. The magnitude of the pressure has two patterns, that is, a case where a constant pressure is always maintained and a case where the pressure is automatically increased according to apnea, and is set by a doctor according to the medical condition of a patient.

Regarding the CPAP apparatus, there is known a technique capable of accurately detecting the pressure of air to be detected (for example, refer to PTL 1). In this technique, a pressure sensor is provided within the CPAP apparatus, has a first port through which air discharged from ae discharge port of the CPAP apparatus is guided, and a second port as an open port, and detects the pressure of the air discharged from the discharge port. An opening is provided in a housing of the CPAP apparatus and communicates with the outside of the CPAP apparatus. A tube connects the opening and the second port of the pressure sensor to each other.

### Citation List

### Patent Literature

[PTL 1] Japanese Unexamined Patent Application, First Publication No. 2018-78997

### Summary of Invention

### Technical Problem

The CPAP apparatus controls the pressurized air so as to be sent into the airway from the nose when the patient is inhaling air and controls the pressure so as to be decreased when the patient is exhaling air. However, in a case where the patient is exhaling air, there is a case where the control of decreasing the pressure may not catch up. In this case, since excessive air continues to be supplied to the patient while exhaling air, there is a case where the patient has difficulty in breathing.

The present invention has been made in view of the above circumstances, and an object thereof is to provide a CPAP system and a CPAP apparatus that can improve difficulty in breathing of a patient using CPAP.

### Solution to Problem

(1) A CPAP system according to an aspect of the present invention includes a fan that suctions air and delivers the air; a sensor that measures a pressure or flow rate of the air delivered by the fan; a case having the fan and the sensor built therein and having an air inflow port that allows the air sent into the fan to flow in therethrough and an air outflow port that allows the air delivered from the fan to flow out therethrough; a motor drive unit that controls a rotation speed of a motor, which rotates the fan on the basis of the pressure or the flow rate measured by the sensor, by a PWM signal; a valve that regulates a pressure or flow rate of air supplied from the air outflow port via a tube to a mask worn on a patient; and a valve drive unit that opens and closes the valve on the basis of the PWM signal. The valve drive unit includes a synthesis unit that synthesizes three-phase signals for driving the motor, on the basis of the PWM signal, and a control unit that controls a closing speed of the valve and an opening degree of the valve. The valve drive unit opens and closes the valve on the basis of a synthesized signal obtained by the synthesis unit synthesizing the three-phase signals, and the closing speed and the opening degree of the valve controlled by the control unit.
(2) In the CPAP system according to the aspect of the present invention, the control unit may control an opening speed of the valve.
(3) In the CPAP system according to the aspect of the present invention, the valve drive unit may include a determination unit that determines the rotation speed of the motor on the basis of a duty ratio of the synthesized signal, and the valve drive unit may open and close the valve on the basis of a result obtained by the determination unit determining the rotation speed of the motor.
(4) In the CPAP system according to the aspect of the present invention, the determination unit may determine that the motor is accelerating in a case where a value obtained by converting the duty ratio of the synthesized signal into a voltage is equal to or higher than a threshold value, and determine that the motor is decelerating in a case where a value obtained by converting the duty ratio of the synthesized signal into a voltage is less than the threshold value.
(5) In the CPAP system according to the aspect of the present invention, the control unit may generate a control signal for opening and closing the valve on the basis of the opening degree and the opening and closing speed of the valve.
(6) A CPAP apparatus according to an aspect of the present invention includes a fan that suctions air and delivers the air; a sensor that measures a pressure or flow rate of the air delivered by the fan; a case having the fan and the sensor built therein and having an air inflow port that allows the air sent into the fan to flow in therethrough and an air outflow port that allows the air delivered from the fan to flow out therethrough; a motor drive unit that controls a rotation speed of a motor, which rotates the fan on the basis of the pressure or the flow rate measured by the sensor, by a PWM signal; a valve that regulates a pressure or flow rate of air supplied from the air outflow port via a tube to a mask worn on a patient; and a valve drive unit that opens and closes the valve on the basis of the PWM signal. The valve drive unit includes a synthesis unit that synthesizes three-phase signals for driving the motor, on the basis of the PWM signal, and a control unit that controls a closing speed of the valve and an opening degree of the valve. The valve drive unit opens and closes the valve on the basis of a synthesized signal obtained by the synthesis unit synthesizing the three-phase signals, and the closing speed and the opening degree of the valve controlled by the control unit.
(7) In the CPAP system according to an aspect of the present invention, the valve includes a first fixing member that is disposed on an outer peripheral side of the tube and combined with the tube; a second fixing member that is combined with the first fixing member in a state where an accommodation space is formed between the first fixing member and the second fixing member; and a movable plate that is disposed in the accommodation space and is movable relative to the first fixing member and the second fixing member. An inside of the accommodation space communicates with an inside of the tube through a first air discharge hole formed in the tube. A second air discharge hole communicating with the outside is formed in the second fixing member. The movable plate is disposed so as to allow closing of the second air discharge hole, and has at least one air communication hole that allows communication between the inside of the second air discharge hole and the inside of the accommodation space. The movable plate may be reciprocable between a fully closed position where the second air discharge hole is fully closed and a fully open position where the second air discharge hole is fully opened through the at least one air communication hole.
(8) In the CPAP system according to the aspect of the present invention, the movable plate may gradually increase an opening degree of the second air discharge hole through the air communication hole as the movable plate moves from the fully closed position to the fully open position.
(9) In the CPAP system according to the aspect of the present invention, a plurality of the air communication holes may be formed so as to be lined up along a movement direction of the movable plate, and may be formed so as to have different opening areas. The movable plate is movable from the fully closed position toward the fully open position such that the plurality of air communication holes sequentially communicate with the second air discharge hole. The second air discharge hole may change in opening degree depending on communication with each of the plurality of air communication holes, and may be fully opened by communication with the air communication hole having a largest opening area when the movable plate is located at the fully open position.
(10) In the CPAP system according to the aspect of the present invention, the movable plate may be reciprocally rotatable between the fully closed position and the fully open position around a rotation axis.
(11) In the CPAP system according to one aspect of the present invention, a guide groove may be formed in the movable plate so as to extend in a circumferential direction around the rotation axis. At least one of the first fixing member and the second fixing member may be formed with a guide projection that is inserted into the guide groove.
(12) In the CPAP apparatus according to the aspect of the present invention, the valve includes a first fixing member that is disposed on an outer peripheral side of the tube and combined with the tube; a second fixing member that is combined with the first fixing member in a state where an accommodation space is formed between the first fixing member and the second fixing member; and a movable plate that is disposed in the accommodation space and is movable relative to the first fixing member and the second fixing member. An inside of the accommodation space communicates with an inside of the tube through a first air discharge hole formed in the tube. A second air discharge hole communicating with the outside is formed in the second fixing member. The movable plate is disposed so as to allow closing of the second air discharge hole, and has at least one air communication hole that allows communication between the inside of the second air discharge hole and the inside of the accommodation space. The movable plate may be reciprocable between a fully closed position where the second air discharge hole is fully closed and a fully open position where the second air discharge hole is fully opened through the at least one air communication hole.

### Advantageous Effects of Invention

According to the present invention, the CPAP system and CPAP apparatus that can improve the difficulty in breathing of the patient using CPAP can be provided.

### Brief Description of Drawings

FIG. 1 is a view illustrating an example of a CPAP system of an embodiment of the present invention.
FIG. 2 is a view illustrating an example of characteristics showing a relationship between a flow rate of air delivered by a CPAP apparatus and a static pressure, with a rotation speed of a fan as a parameter.
FIG. 3 is a view illustrating an example of a characteristic diagram showing a response time.
FIG. 4 is a view illustrating an example of the CPAP apparatus of the embodiment of the present invention.
FIG. 5 is a view illustrating an example of information indicating the rotation speed.
FIG. 6 is a view illustrating an example of a characteristic diagram showing the response time of the CPAP system of the embodiment of the present invention.
FIG. 7 is a block diagram illustrating a valve drive unit of the CPAP apparatus of the embodiment of the present invention.
FIG. 8 is a view illustrating an example of the operation of the valve drive unit of the CPAP apparatus of the embodiment of the present invention.
FIG. 9 is a view illustrating an example of an acceleration signal and a deceleration signal.
FIG. 10 is a view illustrating Example 1 of the operation of the CPAP apparatus of the embodiment of the present invention.
FIG. 11 is a view illustrating Example 2 of the operation of the CPAP apparatus of the embodiment of the present invention.
FIG. 12 is a view illustrating an example of a valve control signal.
FIG. 13 is a view illustrating an example of valve driving.
FIG. 14 is a flowchart illustrating an example of the operation of the CPAP system of the embodiment of the present invention.
FIG. 15 is a view illustrating Example 1 of effects of the CPAP system of the embodiment of the present invention.
FIG. 16 is a view illustrating Example 2 of effects of the CPAP system of the embodiment of the present invention.
FIG. 17 is a view illustrating an example of the operation of the valve drive unit of the CPAP apparatus of the embodiment of the present invention.
FIG. 18 is a view illustrating an example of the operation of the valve drive unit of the CPAP apparatus of the embodiment of the present invention.
FIG. 19 is a view illustrating an example of a CPAP apparatus of a modification example of the embodiment of the present invention.
FIG. 20 is a flowchart illustrating an example of the operation of the CPAP system of the modification example of the embodiment of the present invention.
FIG. 21 is a view illustrating a modification example of a valve in the embodiment of the present invention, and is a perspective view in which the valve is combined with a tube.
FIG. 22 is a perspective view of the valve illustrated in FIG. 21.
FIG. 23 is an exploded perspective view of the valve illustrated in FIG. 22.
FIG. 24 is an exploded perspective view of the valve illustrated in FIG. 22.
FIG. 25 is a perspective view of the valve illustrated in FIG. 22 viewed from below.
FIG. 26 is a perspective view illustrating a state in which a drive motor is combined with a base plate illustrated in FIG. 23.
FIG. 27 is a partial sectional view of the valve illustrating a mounted state of a rotary plate illustrated in FIG. 23.
FIG. 28 is a view illustrating a state in which the rotary plate is combined with the base plate illustrated in FIG. 26, and is a perspective view in which the rotary plate is located at a fully open position.
FIG. 29 is a perspective view illustrating a state where the rotary plate is located at a fully closed position from the state illustrated in FIG. 28.
FIG. 30 is a perspective view illustrating an intermediate stage in which the rotary plate is rotated toward the fully open position from the state illustrated in FIG. 28.
FIG. 31 is a view illustrating a state in which the rotary plate is combined with the base plate illustrated in FIG. 26, and is a perspective view in which the rotary plate is located at a reference position.
FIG. 32 is a plan view illustrating a modification example of the rotary plate illustrated in FIG. 23.
FIG. 33 is a plan view illustrating another modification example of the rotary plate illustrated in FIG. 23.

### Description of Embodiments

Next, a CPAP system and a CPAP apparatus of the present embodiment will be described with reference to the drawings. Embodiments to be described below are merely examples, and the embodiments to which the present invention is applied are not limited to the following embodiments.

The expression "on the basis of XX" referred to in the present application means "on the basis of at least XX" and also includes a case based on another element in addition to XX. The expression "on the basis of XX" is not limited to a case where XX is directly used, and also includes a case where calculation or processing is performed on XX. "XX" is an optional element (for example, optional information).

### (Embodiment)

Hereinafter, embodiments of the present invention will be described with reference to the drawings. There are cases where components having the same or similar functions will be denoted by the same reference signs, and redundant description regarding the components will be omitted.

### (Outline of CPAP system)

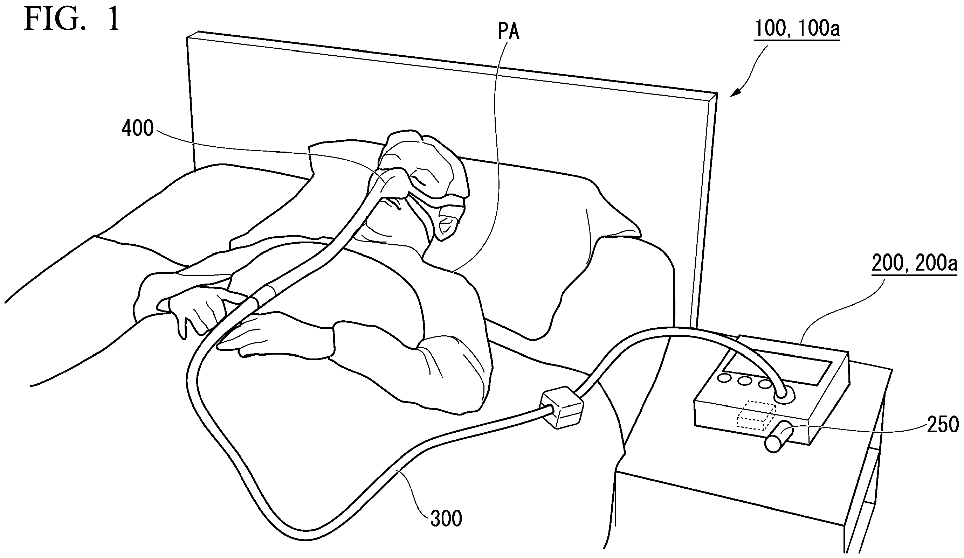

FIG. 1 is a view illustrating an example of a CPAP system of an embodiment of the present invention.

The CPAP system 100 includes a CPAP apparatus 200, a tube 300, and a mask 400.

The CPAP apparatus 200 includes a case having an air inflow port that allows air to flow in therethrough and an air outflow port that allows air to flow out therethrough, a fan, a pressure sensor, and a valve 250. The fan and the pressure sensor are built in the case.

The fan suctions air into the CPAP apparatus 200 from the air inflow port, and delivers the suctioned air to the tube 300 connected to the air outflow port of the CPAP apparatus 200.

The pressure sensor measures the pressure of the air delivered to the CPAP apparatus 200 by the fan.

The valve 250 regulates the pressure of the air supplied from the air outflow port via the tube 300 to the mask 400.

The tube 300 is connected to the air outflow port of the CPAP apparatus 200 and delivers the air delivered by the fan to the mask 400.

The mask 400 is connected to the tube 300 and worn on a patient PA. The mask 400 delivers the air delivered by the CPAP apparatus 200.

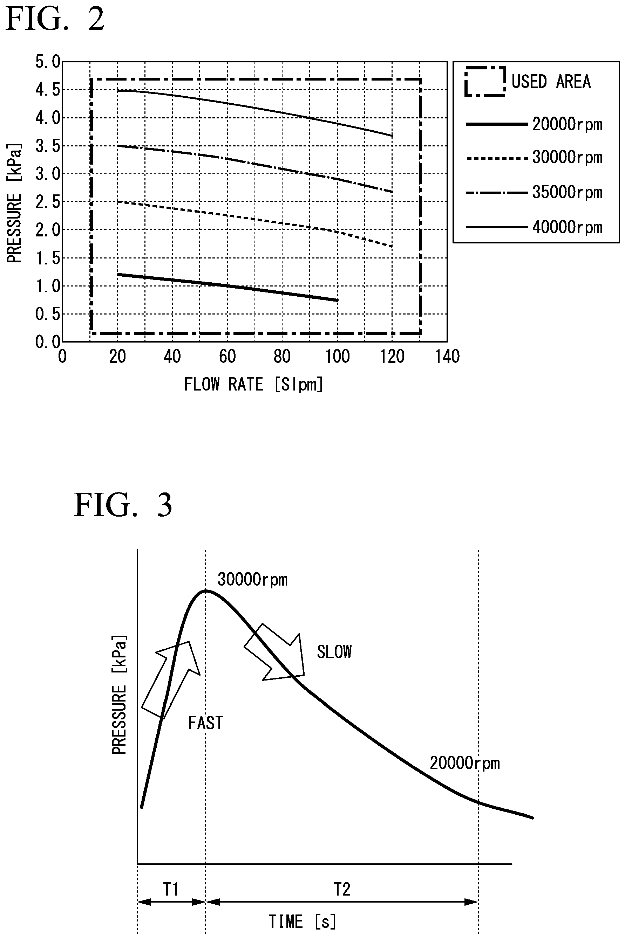

FIG. 2 is a view illustrating an example of a characteristic showing a relationship between the flow rate of the air delivered by the CPAP apparatus and the static pressure with the rotation speed of the fan as a parameter. In FIG. 2, the horizontal axis represents the flow rate [slpm] of the air delivered by the CPAP apparatus 200, and the vertical axis represents the pressure [kPa].

FIG. 2 illustrates a case where the rotation speed of the fan is changed to 20000 r/min, 25000 r/min, 30000 r/min, 35000 r/min, and 40000 r/min. The higher the rotation speed of the fan, the higher the obtained pressure (static pressure).

In FIG. 2, an example of a region to be used is indicated by a dashed line. The region used herein is expressed by a range of the flow rate and a range of the pressure. Specifically, the range of the flow rate to be used is about 10 [slpm] to 140 [slpm], and the range of the pressure to be used is about 0.3 [kPa] to 4.5 [kPa].

The pressure obtained in a case where when the flow rate of the air delivered from the CPAP apparatus 200 is 20 [slpm] to 100 [slpm] and the rotation speed of the fan is 20000 [r/min] is about 0.6 [kPa] to 1.1 [kPa].

The pressure obtained in a case where the flow rate of the air delivered from the CPAP apparatus 200 is 20 [slpm] to 100 [slpm] and the rotation speed of the fan is 25000 [r/min] is about 1.2 [kPa] to 1.8 [kPa].

The pressure obtained in a case where the flow rate of the air delivered from the CPAP apparatus 200 is 20 [slpm] to 120 [slpm] and the rotation speed of the fan is 30000 [r/min] is about 1.8 [kPa] to 2.5 [kPa].

The pressure obtained in a case where the flow rate of the air delivered from the CPAP apparatus 200 is 20 [slpm] to 140 [slpm] and the rotation speed of the fan is 35000 [r/min] is about 2.5 [kPa] to 3.5 [kPa].

The pressure obtained in a case where the flow rate of the air delivered by the CPAP apparatus 200 is 30 [slpm] to 110 [slpm] and the rotation speed of the fan is 40000 [r/min] is about 3.9 [kPa] to 4.5 [kPa].

Here, a case where it is assumed that the pressure is adjusted a value between 0.5 [kPa] and 2.5 [kPa] by adjusting the rotation speed of the fan between 20000 r/min and 35000 r/min at a flow rate of 125 [slpm] will be described.

In other words, when the patient is inhaling air, the rotation speed of the fan is increased from 20000 [r/min] to 35000 [r/min] such that the pressurized air is sent into the airway from the nose, and when the patient exhales the air, the rotation speed of the fan is reduced from 35000 [r/min] to 20000 [r/min] in order to decrease the pressure.

FIG. 3 is a view illustrating an example of a characteristic diagram showing a response time. In the example illustrated in FIG. 3, an example of the response time in a case where the pressure is adjusted between 0.5 [kPa] and 2.5 [kPa] by adjusting the rotation speed of the fan between 20000 r/min and 35000 r/min at a flow rate of 125 [slpm] is illustrated.

The time required to increase the rotation speed of the fan from 20000 r/min to 35000 r/min is defined as a rotation speed increase response time T1, and the time required to reduce the rotation speed of the fan from 35000 r/min to 20000 r/min is defined as a rotation speed reduction response time T2.

The fan is rotated by a DC motor. The DC motor has a slower fall time for which the rotation speed is reduced than a rise time for which the rotation speed is increased. For this reason, the rotation speed increase response time T1 is shorter than the rotation speed reduction response time T2.

In a case where the pressure of the air supplied from the air outflow port via the tube 300 to the mask 400 is not regulated by the valve 250, even if the rotation speed of the fan is reduced from 35000 [r/min] to 20000 [r/min] in order to decrease the pressure when the patient is exhaling air, a decrease in pressure is slow and a substantial time is required for the decrease in pressure because the rotation speed reduction response time T2 is long. Since a substantial time is required for the decrease in pressure, the pressure remains when the patient PA is exhaling air, and the air having a pressure higher than necessary continues being applied. For this reason, there is a case where the patient PA has difficulty in breathing.

Thus, the CPAP system 100 of the embodiment regulates the pressure of the air supplied from the air outflow port via the tube 300 to the mask 400 by opening the valve 250 in a case where the patient PA is exhaling air. With such a configuration, in a case where the patient PA is exhaling air, the pressure of the air applied to the patient PA can be decreased. Therefore, it is possible to improve the difficulty in breathing of the patient PA.

Moreover, the CPAP system 100 of the embodiment adjusts the opening degree of the valve 250 and the speed at which the valve 250 is closed up to the opening degree such that the valve 250 is slowly closed in a case where the open valve 250 is closed. With such a configuration, it is possible to prevent the pressure of the tube 300 from changing sharply by opening the valve 250 and then rapidly closing the valve 250. For this reason, it is possible to improve the difficulty in breathing of the patient PA.

In addition, the CPAP system 100 of the embodiment adjusts the opening degree of the valve 250 and the speed at which the valve 250 is opened up to the opening degree such that the valve 250 opens slowly in a case where the closed valve 250 is opened.

Hereinafter, the CPAP apparatus 200 included in the CPAP system 100 will be described in detail.

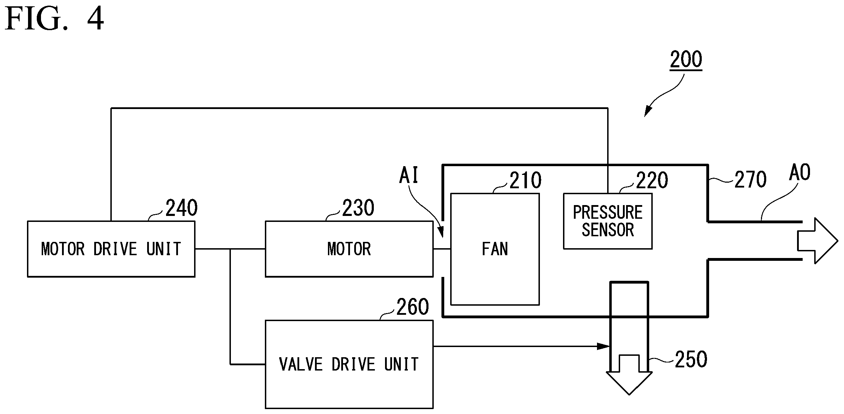

FIG. 4 is a view illustrating an example of the CPAP apparatus of the embodiment of the present invention.

As illustrated in FIG. 4, the CPAP apparatus 200 includes a fan 210, a pressure sensor 220, a motor 230, a motor drive unit 240, a valve 250, a valve drive unit 260, and a case 270.

The fan 210 is built in the case 270, suctions air from an air inflow port AI of the case 270, and delivers the suctioned air to an air outflow port AO.

The pressure sensor 220 is built in the case 270 and regularly measures the pressure of the air delivered by the fan 210. The pressure sensor 220 regularly outputs the measurement result of the pressure of air to the motor drive unit 240.

The motor 230 is connected to the fan 210 and rotates the fan 210 on the basis of the information indicating the rotation speed output by the motor drive unit 240. An example of the motor 230 is a three-phase induction motor (three-phase motor). Hereinafter, a case where the three-phase induction motor is applied as an example of the motor 230 will continue being described.

The motor drive unit 240 acquires the measurement result of the pressure of air output by the pressure sensor 220. The motor drive unit 240 determines the rotation speed set for the motor 230 on the basis of the acquired measurement result of the pressure of air. The motor drive unit 240 outputs information indicating the rotation speed to the motor 230 on the basis of the determined rotation speed. The information indicating the rotation speed output by the motor drive unit 240 is also output to the valve drive unit 260. An example of the information indicating the rotation speed output by the motor drive unit 240 is a pulse width modulation (PWM) signal. Hereinafter, a case where the PWM signal is applied as the information indicating the rotation speed will continue being described.

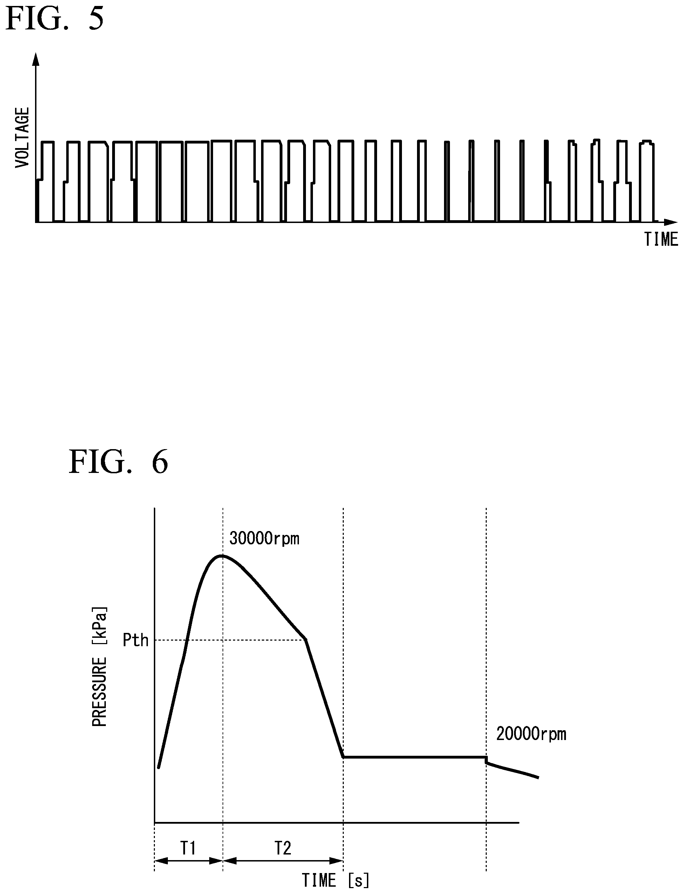

FIG. 5 is a view illustrating an example of the information indicating the rotation speed.

In a case where the measurement result of the pressure of air has been reduced due to the patient PA inhaling air, the motor drive unit 240 increases the duty ratio of the PWM signal on the basis of the acquired measurement result of the pressure of air. Since the rotation speed of the fan 210 can be increased by increasing the duty ratio of the PWM signal, the pressurized air can be sent into the airway from the nose for the patient PA who is inhaling the air.

On the other hand, the motor drive unit 240 reduces the duty ratio on the basis of the acquired measurement result of the pressure of air in a case where the measurement result of the pressure of air has increased due to the patient PA exhaling air. Since the rotation speed of the fan 210 can be reduced by reducing the duty ratio, the pressure of the air supplied to the mask 400 can be decreased for the patient PA who is exhaling air.

FIG. 5 illustrates an example in which the duty ratio is increased and then reduced. Returning to FIG. 4, the description will be continued.

The valve drive unit 260 acquires the PWM signal output by the motor drive unit 240. The valve drive unit 260 generates a pulse (hereinafter, referred to as "driving pulse") for driving the valve 250 on the basis of the acquired PWM signal.

Specifically, the valve drive unit 260 synthesizes a three-phase signal that drives the motor 230 that rotates the fan 210, on the basis of the acquired PWM signal. The valve drive unit 260 determines whether or not the motor 230 is accelerating or decelerating on the basis of the duty ratio of the synthesized signal obtained by synthesizing the three-phase signals.

In a case where it is determined that the motor 230 is accelerating, on the basis of the determination result of whether or not the motor 230 is accelerating or decelerating, the valve drive unit 260 generate a pulse for slowly closing the valve 250 (hereinafter referred to as "closed pulse"). This is because it is conceived that the pressure is first reduced by the patient PA inhaling the air and the fan is supplying air to assist in the inhalation. An example of the closed pulse output by the valve drive unit 260 is a PWM signal.

In this way, in a case where the pressure is first reduced by the patient PA inhaling air and the fan is supplying air to assist in the inhalation, the valve drive unit 260 generates a closed pulse for slowly closing the valve 250, thereby slowly closing the valve 250. With such a configuration, it is possible to prevent the pressure in the tube 300 or the like from changing sharply. In addition, since it is possible to prevent air from flowing out from the valve 250, it is possible to prevent the pressure from decreasing.

In a case where it is determined that the motor 230 is decelerating on the basis of the determination result of whether or not the motor 230 is accelerating or decelerating, the valve drive unit 260 generate a pulse for slowly opening the valve 250 (hereinafter referred to as "an open pulse"). This is because it is conceived that the pressure has increased due to the patient PA exhaling air. An example of the open pulse output by the valve drive unit 260 is a PWM signal.

In this way, in a case where the pressure has increased due to the patient PA exhaling air, the valve drive unit 260 slowly open the valve 250 by generating the open pulse for slowly opening the valve 250. With such a configuration, since the air can slowly flow out of the valve 250, the pressure can be slowly decreased.

The valve 250 is built in the case 270 and regulates the pressure of the air supplied from the air outflow port AO via the tube 300 connected to the air outflow port AO to the mask 400 worn on the patient PA.

Examples of the valve 250 are an electromagnetic valve, a linear valve, and a wastegate valve. Hereinafter, the description will continue being described regarding a case where the valve 250 is the linear valve. Specifically, the valve 250 regulates the pressure of the air supplied from the air outflow port AO via to the tube 300 the mask 400 worn on the patient PA by slowly opening and closing the valve 250 in accordance with the driving pulse output by the valve drive unit 260.

In a case where the valve 250 has acquired the closed pulse output by the valve drive unit 260, the valve 250 is slowly closed on the basis of the acquired closed pulse. In this way, since the valve 250 can prevent air from flowing out from the valve 250 by being slowly closed on the basis of the closed pulse output d by the valve drive unit 260, the pressure can be prevented from increasing sharply.

On the other hand, in a case where the valve 250 has acquired the open pulse output by the valve drive unit 260, the valve 250 is slowly opened on the basis of the acquired open pulse. In this way, since the valve 250 can allow air to flow out of the valve 250 by being slowly opened on the basis of the open pulse output by the valve drive unit 260, the pressure can be slowly decreased.

FIG. 6 is a view illustrating an example of a characteristic diagram showing the response time of the CPAP system of the embodiment of the present invention.

In the example illustrated in FIG. 6, similarly to the example illustrated in FIG. 3, an example of the response time in a case where the pressure is adjusted between 0.5 [kPa] and 2.5 [kPa] by adjusting the rotation speed of the fan between 20000 r/min and 35000 r/min at a flow rate of 125 [slpm] is illustrated.

It can be seen that, compared to FIG. 3, the rotation speed increase response time T1 is about the same, but the rotation speed reduction response time T2 is shortened. This is because, in the present embodiment, in a case where it is determined that the motor 230 is decelerating due to an increase in pressure, the valve drive unit 260 generates an open pulse, so that air leaks from the valve 250 and the pressure within the CPAP apparatus 200 decreases faster than in a case where the valve is not opened. On the other hand, the speed at which the pressure decreases slowly because the valve 250 opens slowly.

The aforementioned valve drive unit 260 will be described in detail.

### (Details of Valve Drive Unit)

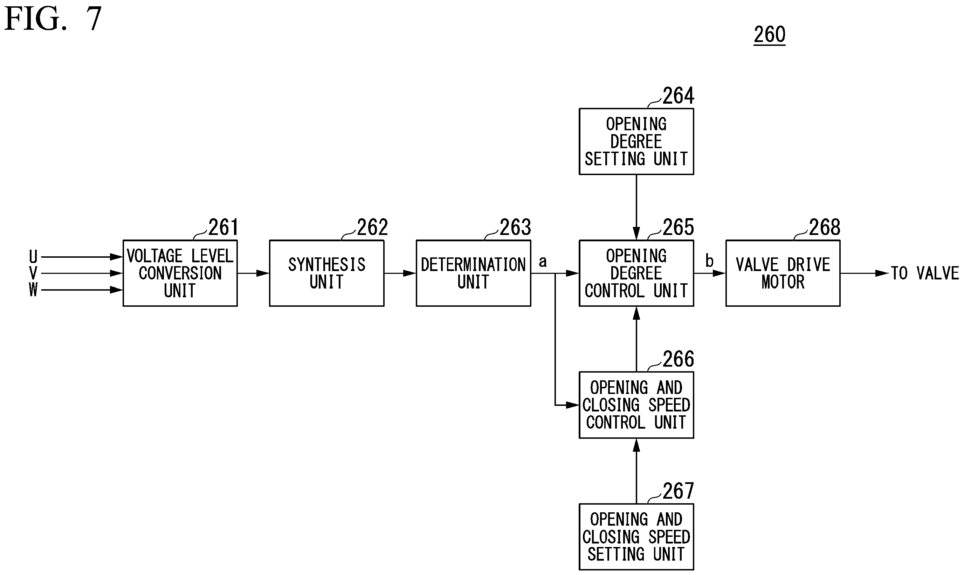

FIG. 7 is a block diagram illustrating the valve drive unit of the CPAP apparatus of the embodiment of the present invention.

The valve drive unit 260 of the CPAP apparatus 200 includes a voltage level conversion unit 261, a synthesis unit 262, a determination unit 263, an opening degree setting unit 264, an opening degree control unit 265, an opening and closing speed control unit 266, and an opening and closing speed setting unit 267, and a valve drive motor 268.

The valve drive unit 260 is constituted by the voltage level conversion unit 261, the synthesis unit 262, the determination unit 263, the opening degree setting unit 264, the opening degree control unit 265, the opening and closing speed control unit 266, the opening and closing speed setting unit 267, and the valve drive motor 268. Accordingly, the circuit configuration can be simplified and the propagation delay can be reduced. Since the operation speed can be improved by reducing the propagation delay, the respiratory response of CPAP can be improved. In addition, since the circuit configuration can be simplified, the apparatus can be downsized and the power consumption can be reduced due to the area reduction effect.

Each of a U-phase output voltage, a V-phase output voltage, and a W-phase output voltage, which are the PWM signals output by the motor drive unit 240, is supplied to the motor 230.

Specifically, the U-phase output voltage is supplied to a U-phase coil of the motor 230, the V-phase output voltage is supplied to a V-phase coil of the motor 230, and the W-phase output voltage is supplied to a W-phase coil of the motor 230. Moreover, each of the U-phase output voltage, the V-phase output voltage, and the W-phase output voltage, which are the PWM signals output by the motor drive unit 240, is output to the voltage level conversion unit 261 of the valve drive unit 260.

The voltage level conversion unit 261 steps down the voltage level of each of the U-phase output voltage, the V-phase output voltage, and the W-phase output voltage output by the motor drive unit 240 to a low voltage such as 3 V or 5 V, and outputs each of the U-phase output voltage, the V-phase output voltage, and the W-phase output voltage with the stepped-down voltage levels to the synthesis unit 262.

Each of the U-phase output voltage, the V-phase output voltage, and the W-phase output voltage output by the motor drive unit 240 can be adjusted so as to be output to the subsequent stage by stepping down the voltage level of each of the U-phase output voltage, the V-phase output voltage, and the W-phase output voltage, respectively. In addition, since a low-voltage-resistant circuit can be used and configured by stepping down the voltage level of each of the U-phase output voltage, the V-phase output voltage, and the W-phase output voltage, the voltage level conversion unit 261 can be realized at low cost.

Hereinafter, as an example, a case where the voltage level of each of the U-phase output voltage, the V-phase output voltage, and the W-phase output voltage are stepped down to 5 V will continue being described.

The synthesis unit 262 acquires each of the U-phase output voltage, the V-phase output voltage, and the W-phase output voltage with the stepped-down voltage levels that are output by the voltage level conversion unit 261, and synthesizes the acquired U-phase output voltage, V-phase output voltage, and W-phase output voltage with the stepped-down voltage levels.

Specifically, the synthesis unit 262 logically synthesizes the acquired U-phase output voltage, V-phase output voltage, and W-phase output voltage whose voltage levels have been stepped down, thereby synthesizing the U-phase output voltage, the V-phase output voltage, and the W-phase output voltage with the stepped-down voltage levels. The synthesis unit 262 outputs, to the determination unit 263, a synthesized signal obtained by synthesizing each of the U-phase output voltage, the V-phase output voltage, and the W-phase output voltage with the stepped-down voltage levels.

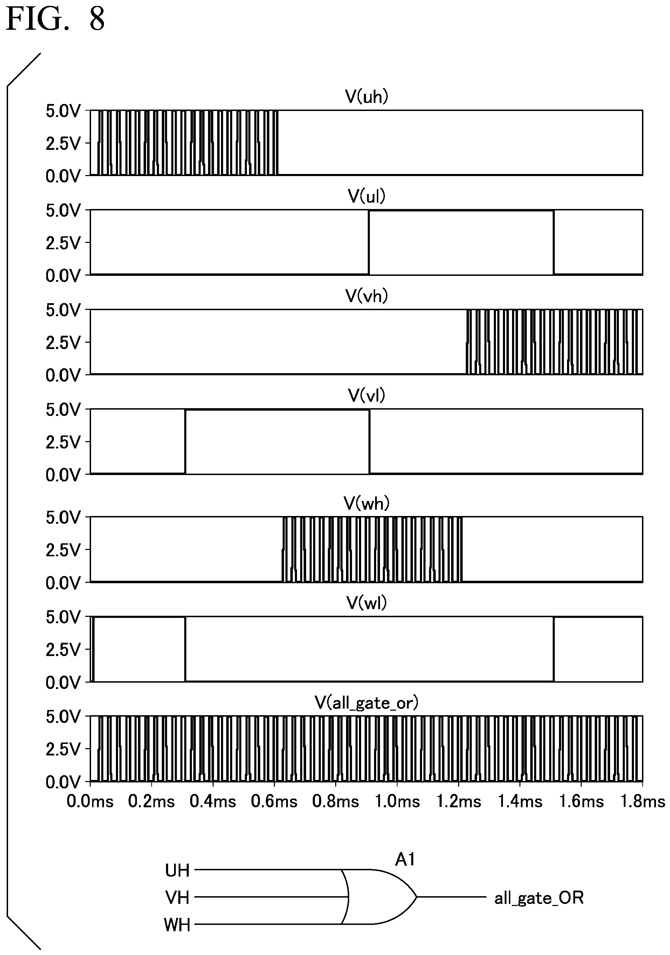

FIG. 8 is a view illustrating an example of the operation of the valve drive unit of the CPAP apparatus of the embodiment of the present invention. An upper part of FIG. 8 illustrates that a rectangular signal is obtained by synthesizing the U-phase output voltage (V (uh)), the V-phase output voltage (V (vh)), and the W-phase output voltage (V (wh)) with the stepped-down voltage levels output by the voltage level conversion unit 261.

A lower part of FIG. 8 illustrates that the synthesized signal is expressed by a logical sum of the U-phase output voltage (V (uh)), the V-phase output voltage (V (vh)), and the W-phase output voltage (V (wh)). Returning to FIG. 7, the description will be continued.

The determination unit 263 acquires the synthesized signal output by the synthesis unit 262, and determines the rotation speed of the motor 230 on the basis of the duty ratio of the acquired synthesized signal.

Specifically, the determination unit 263 determines that the motor 230 is accelerating in a case where a value obtained by converting the frequency or duty ratio of the synthesized signal into a voltage is equal to or higher than a threshold value, and determines that the motor 230 is decelerating in a case where the value obtained by converting the frequency or duty ratio of the synthesized signal into the voltage is less than the threshold value. By determining whether the motor 230 is accelerating or decelerating, it is possible to detect whether the state of the patient PA is inhaling or exhaling.

In a case where the determination unit 263 determines that the motor 230 is accelerating, the determination unit 263 outputs a signal indicating that the motor 230 is accelerating (hereinafter referred to as an "acceleration signal") to the opening degree control unit 265 and the opening and closing speed control unit 266. In a case where the determination unit 263 determines that the motor 230 is decelerating, the determination unit 263 outputs a signal indicating that the motor 230 is decelerating (hereinafter referred to as a "deceleration signal") to the opening degree control unit 265 and the opening and closing speed control unit 266.

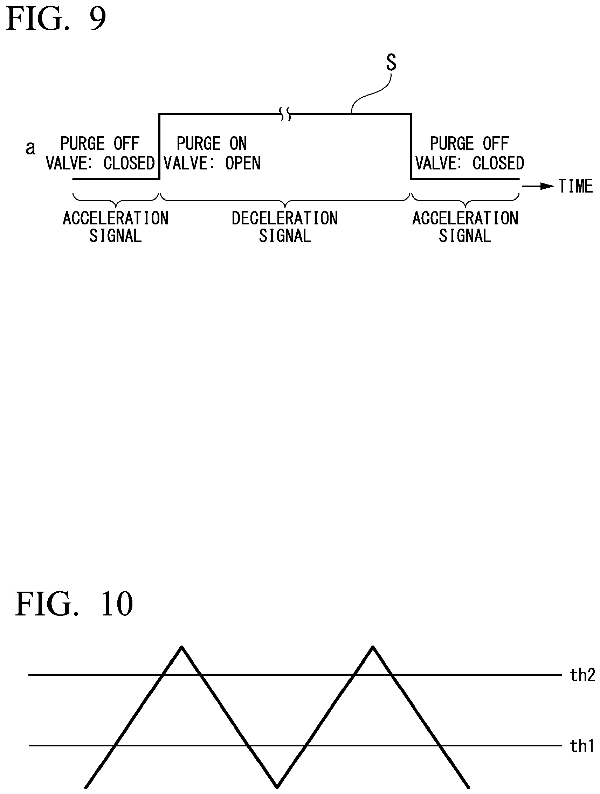

FIG. 9 is a view illustrating an example of the acceleration signal and the deceleration signal.

As previously mentioned, the determination unit 263 outputs an acceleration and deceleration signal S including the acceleration signal and the deceleration signal. As illustrated in FIG. 9, the acceleration signal corresponds to OFF and the deceleration signal corresponds to ON. Returning to FIG. 7, the description will be continued.

The opening degree setting unit 264 sets the opening degree of the valve 250.

FIG. 10 is a view illustrating Example 1 of the operation of the CPAP apparatus of the embodiment of the present invention. Here, as an example, a case where the opening degree setting unit 264 sets the opening degree of the valve 250 on the basis of a triangular wave will be described. The opening degree setting unit 264 may set the opening degree of the valve 250 on the basis of a waveform other than the triangular wave.

The opening degree setting unit 264 applies one or a plurality of threshold values to the triangular wave. For example, the opening degree setting unit 264 applies a first threshold value th1 and a second threshold value th2, thereby deriving the time width of a triangular wave in a case where the first threshold value th1 is applied and the time width of a triangular wave in a case where the second threshold value th2 is applied. The opening degree setting unit 264 converts a triangular wave into a rectangular wave by setting the first threshold value, turning on the set first threshold value or higher and turning off less than the first threshold value, and derives the time width on the basis of the rectangular wave obtained by the conversion.

Thereafter, the opening degree setting unit 264 converts the triangular wave into a rectangular wave by setting the second threshold value, turning on the set second threshold value or higher, and turning off less than the second threshold value, and derives the time width on the basis of the rectangular wave obtained by the conversion. The second threshold value may be a value equal to or greater than the first threshold value or a value less than the first threshold value.

The opening degree setting unit 264 derives the opening degree of the valve 250 on the basis of the derived time width.

Specifically, in a case where the time width derived by the opening degree setting unit 264 is t1 [ms], the opening degree that can be reached at t1 [ms] is derived. The opening degree setting unit 264 may include information in a table format in which the time width and the reachable opening degree [%] are associated with each other.

The opening degree setting unit 264 derives the opening degree of the valve 250 on the basis of the information in the table format, and outputs information indicating the derived opening degree of the valve 250 to the opening degree control unit 265. Returning to FIG. 7, the description will be continued.

The opening and closing speed setting unit 267 derives the time for setting the opening and closing speed.

Specifically, the opening and closing speed setting unit 267 derives the time for setting the opening and closing speed on the basis of a time constant. The opening and closing speed setting unit 267 outputs information indicating the derived time for setting the opening and closing speed to the opening and closing speed control unit 266.

The opening and closing speed control unit 266 acquires the acceleration and deceleration signal S output by the determination unit 263. The opening and closing speed control unit 266 acquires information indicating the time for setting the opening and closing speed output by the opening and closing speed setting unit 267. The opening and closing speed control unit 266 sets a timing when the opening degree control unit 265 outputs a valve control signal for controlling the valve 250, on the basis of the acquired information indicating the time for setting the opening and closing speed.

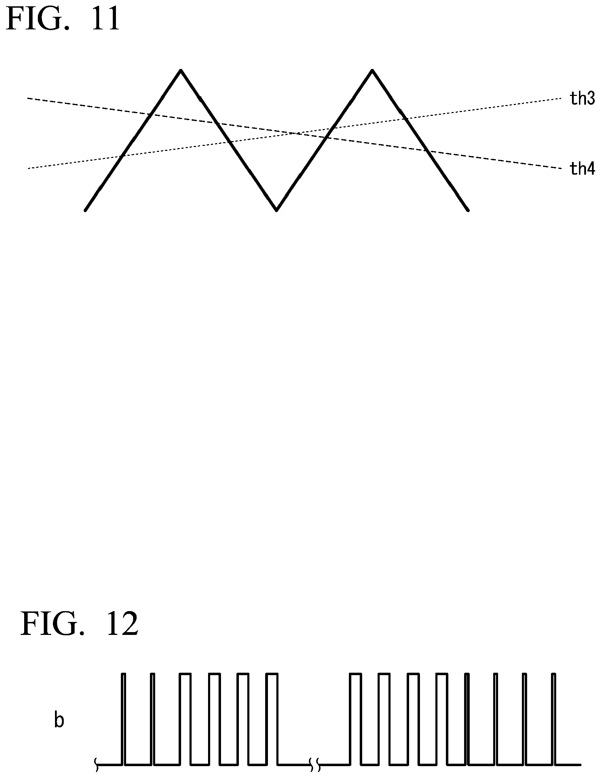

FIG. 11 is a view illustrating Example 2 of the operation of the CPAP apparatus of the embodiment of the present invention.

Here, as an example, a case where the opening and closing speed control unit 266 sets the timing for outputting the valve control signal for controlling the valve 250, on the basis of the triangular wave, will be described. The opening and closing speed control unit 266 may set the timing when the valve control signal for controlling the valve 250 is output, on the basis of a waveform other than the triangular wave. The opening and closing speed control unit 266 applies one or a plurality of threshold values to the triangular wave on the basis of the time for setting the opening and closing speed. Each of one or a plurality of threshold values may be expressed by a decreasing function that decreases with the passage of time or an increasing function that increases with the passage of time.

For example, the opening and closing speed control unit 266 applies a third threshold value th3 and creates timing notification information at a timing when the triangular wave and the third threshold value th3 intersect each other. The opening and closing speed control unit 266 outputs the created timing notification information to the opening degree control unit 265. The opening and closing speed of the valve 250 can be changed by outputting the timing notification information to the opening degree control unit 265 at the timing when the triangular wave and the third threshold value th3 intersect each other.

Thereafter, the opening and closing speed control unit 266 applies a fourth threshold value th4, and derives a timing when the triangular wave and the fourth threshold value th4 intersect each other, thereby deriving the opening and closing speed in a case where the fourth threshold value th4 is applied. The opening and closing speed control unit 266 creates timing notification information at the derived timing when the triangular wave and the fourth threshold value th4 intersect each other. The opening and closing speed control unit 266 outputs the created timing notification information to the opening degree control unit 265.

The opening and closing speed control unit 266 can change the opening and closing speed of the valve 250 by outputting the timing notification information to the opening degree control unit 265 at the timing when the triangular wave and the fourth threshold value th4 intersect each other. The inclination of the fourth threshold value may be an inclination equal to or higher than the third threshold value, or may be an inclination less than the third threshold value. Returning to FIG. 7, the description will be continued.

The opening degree control unit 265 acquires information indicating the opening degree of the valve 250 output by the opening degree setting unit 264. The opening degree control unit 265 acquires the timing notification information output by the opening and closing speed control unit 266. The opening degree control unit 265 creates the valve control signal for controlling the opening degree of the valve 250 on the basis of the acquired information indicating the opening degree of the valve 250, and outputs the created valve control signal to the valve drive motor 268 on the basis of the acquired timing notification information.

FIG. 12 is a view illustrating an example of the valve control signal.

In the valve control signal illustrated in FIG. 12, the opening degree of the valve 250 is indicated by the width (duty) of ON of the valve control signal. In the valve control signal illustrated in FIG. 12, the opening and closing speed of the valve 250 is expressed by the time for which the width (duty) of ON of the valve control signal changes.

The valve drive motor 268 acquires the valve control signal output by the opening degree control unit 265, and controls the valve 250 on the basis of the acquired valve control signal.

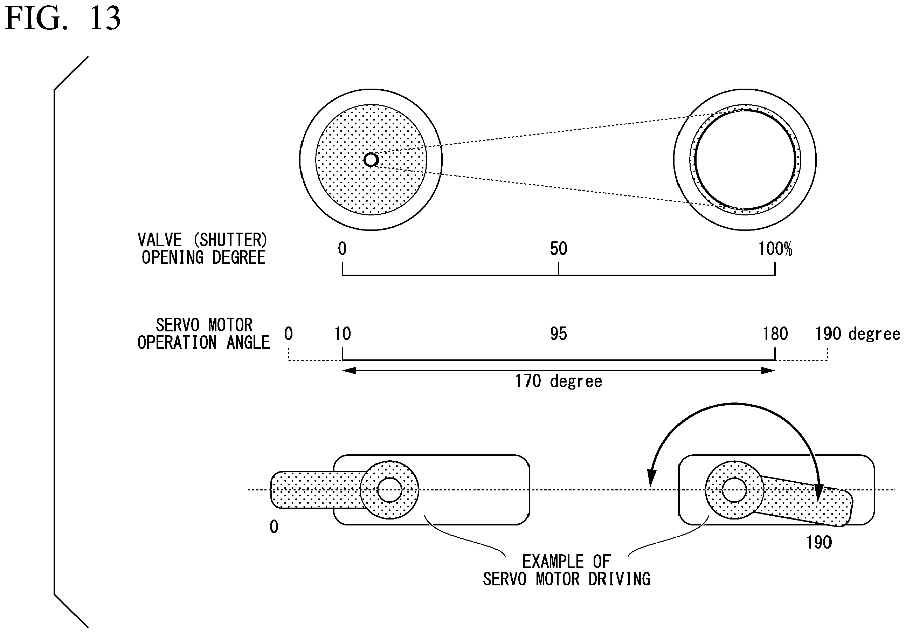

FIG. 13 is a view illustrating an example of valve driving. An example of the valve 250 is a linear valve. Here, a shutter valve will be described as an example of the linear valve. As illustrated in an upper diagram of FIG. 13, the valve 250 can adjust the opening degree between 0% and 100%. Here, as illustrated in a lower diagram of FIG. 13, an opening degree of 0% corresponds to a servo motor operating angle of 0 degrees, and an opening degree of 100% corresponds to a servo motor operating angle of 190 degrees.

The opening degree control unit 265 may control the servo motor operating angle at an angle larger than 0 degree, for example, between 10 degrees and 180 degrees. With such a configuration, since the valve 250 is not completely closed, a flap can be protected at the maximum pressure. In addition, it is possible to prevent a mechanical stress from being applied from a shaft. In addition, since the maximum opening is not made, the mechanical stress from the shaft can be prevented from being applied at the time of opening. In addition, by making the operating angle of the motor as wide as possible with respect to the opening degree of the valve 250 (widening the dynamic range), the resolution of the operating angle becomes high and the control accuracy can be improved.

### (Operation of CPAP system)

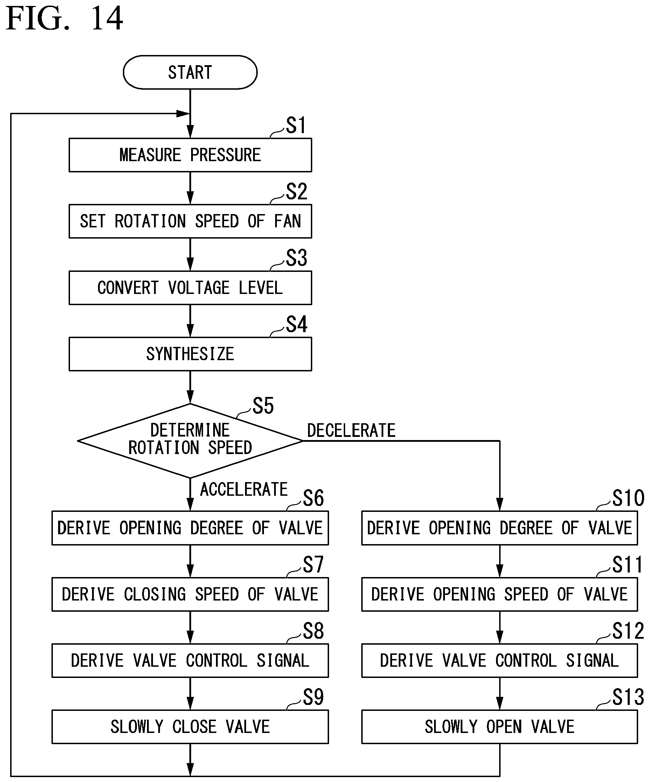

FIG. 14 is a flowchart illustrating an example of the operation of the CPAP system of the embodiment of the present invention. FIG. 14 illustrates the operation after the CPAP system 100 is started. That is, the operation of the CPAP apparatus 200 after the fan 210 is rotated is illustrated.

### (Step S1)

The pressure sensor 220 of the CPAP apparatus 200 measures the pressure of the air delivered by the fan 210. The pressure sensor 220 outputs the measurement result of the pressure of the air to the motor drive unit 240.

### (Step S2)

The motor drive unit 240 of the CPAP apparatus 200 acquires the measurement result of the pressure of the air output by the pressure sensor 220, and determines the rotation speed set for the motor 230 on the basis of the acquired measurement result of the pressure of the air. The motor drive unit 240 outputs the information indicating the rotation speed to the motor 230 and the valve drive unit 260 on the basis of the determined rotation speed. The motor drive unit 240 outputs a PWM signal as the information indicating the rotation speed.

The motor 230 acquires the PWM signal output by the motor drive unit 240 and rotates the fan 210 on the basis of the acquired PWM signal.

### (Step S3)

The PWM signal output by the motor drive unit 240 is also output to the valve drive unit 260.

The voltage level conversion unit 261 of the valve drive unit 260 acquires the U-phase output voltage, the V-phase output voltage, and the W-phase output voltage, which are the PWM signals output by the motor drive unit 240, and steps down the voltage level of each of the acquired U-phase output voltage, V-phase output voltage, and W-phase output voltage. The voltage level conversion unit 261 outputs each of the U-phase output voltage, the V-phase output voltage, and the W-phase output voltage with the stepped-down voltage levels to the synthesis unit 262.

### (Step S4)

The synthesis unit 262 acquires each of the U-phase output voltage, the V-phase output voltage, and the W-phase output voltage with the stepped-down voltage levels that are output by the voltage level conversion unit 261, and synthesizes the acquired U-phase output voltage, V-phase output voltage, and W-phase output voltage with the stepped-down voltage levels. The synthesis unit 262 outputs, to the determination unit 263, a synthesized signal obtained by synthesizing each of the U-phase output voltage, the V-phase output voltage, and the W-phase output voltage with the stepped-down voltage levels.

### (Step S5)

The determination unit 263 acquires the synthesized signal output by the synthesis unit 262, and determines the rotation speed of the motor 230 on the basis of the duty ratio of the acquired synthesized signal. In a case where it is determined that the motor 230 is accelerating, the determination unit 263 outputs an acceleration signal to the opening degree control unit 265 and the opening and closing speed control unit 266. In a case where it is determined that the motor 230 is decelerating, the determination unit 263 outputs a deceleration signal to the opening degree control unit 265 and the opening and closing speed control unit 266.

### (Step S6)

The opening degree setting unit 264 applies one or a plurality of threshold values to the triangular wave, and derives a time width in a case where each of the one or a plurality of threshold values is applied. The opening degree setting unit 264 derives the opening degree of the valve 250 on the basis of the derived time width. The opening degree setting unit 264 outputs the derived information indicating the opening degree of the valve 250 to the opening degree control unit 265.

### (Step S7)

The opening and closing speed control unit 266 acquires the acceleration and deceleration signal S output by the determination unit 263. The opening and closing speed control unit 266 acquires information indicating the time for setting the opening and closing speed output by the opening and closing speed setting unit 267. The opening and closing speed control unit 266 sets the timing when the opening degree control unit 265 outputs the valve control signal for controlling the valve 250, on the basis of the triangular wave and the information indicating the time for setting the opening and closing speed. The opening and closing speed control unit 266 applies a threshold value and creates timing notification information at the timing when the triangular wave and the threshold value intersect each other. The opening and closing speed control unit 266 outputs the created timing notification information to the opening degree control unit 265.

### (Step S8)

The opening degree control unit 265 acquires information indicating the opening degree of the valve 250 output by the opening degree setting unit 264. The opening degree control unit 265 acquires the timing notification information output by the opening and closing speed control unit 266. The opening degree control unit 265 creates the valve control signal for controlling the opening degree of the valve 250 on the basis of the acquired information indicating the opening degree of the valve 250, and outputs the created valve control signal to the valve drive motor 268 on the basis of the acquired timing notification information.

### (Step S9)

The valve drive motor 268 acquires the valve control signal output by the opening degree control unit 265, and controls the valve 250 on the basis of the acquired valve control signal. The valve 250 slowly closes on the basis of the valve control signal output by the valve drive motor 268. By slowly closing the valve 250, the amount of air flowing out from the valve 250 is gradually reduced. Therefore, it is possible to prevent the pressure from rapidly rising in a case where the fan is supplying air to assist in the inhalation.

### (Step S 10)

The opening degree setting unit 264 applies one or a plurality of threshold values to the triangular wave, and derives a time width in a case where each of the one or a plurality of threshold values is applied. The opening degree setting unit 264 derives the opening degree of the valve 250 on the basis of the derived time width of the deceleration signal. The opening degree setting unit 264 outputs the derived information indicating the opening degree of the valve 250 to the opening degree control unit 265.

### (Step S11)

The opening and closing speed control unit 266 acquires the acceleration and deceleration signal S output by the determination unit 263. The opening and closing speed control unit 266 acquires information indicating the time for setting the opening and closing speed output by the opening and closing speed setting unit 267. The opening and closing speed control unit 266 sets the timing when the opening degree control unit 265 outputs the valve control signal for controlling the valve 250, on the basis of the triangular wave and the information indicating the time for setting the opening and closing speed. The opening and closing speed control unit 266 applies a threshold value and creates timing notification information at the timing when the triangular wave and the threshold value intersect each other. The opening and closing speed control unit 266 outputs the created timing notification information to the opening degree control unit 265.

### (Step S 12)

The opening degree control unit 265 acquires information indicating the opening degree of the valve 250 output by the opening degree setting unit 264. The opening degree control unit 265 acquires the timing notification information output by the opening and closing speed control unit 266. The opening degree control unit 265 creates the valve control signal for controlling the opening degree of the valve 250 on the basis of the acquired information indicating the opening degree of the valve 250, and outputs the created valve control signal to the valve drive motor 268 on the basis of the acquired timing notification information.

### (Step S13)

The valve drive motor 268 acquires the valve control signal output by the opening degree control unit 265, and controls the valve 250 on the basis of the acquired valve control signal. The valve 250 opens slowly on the basis of the valve control signal output by the valve drive motor 268. By slowly opening the valve 250, air gradually flows out from the valve 250.

Here, the effects of the CPAP system of the embodiment will be described.

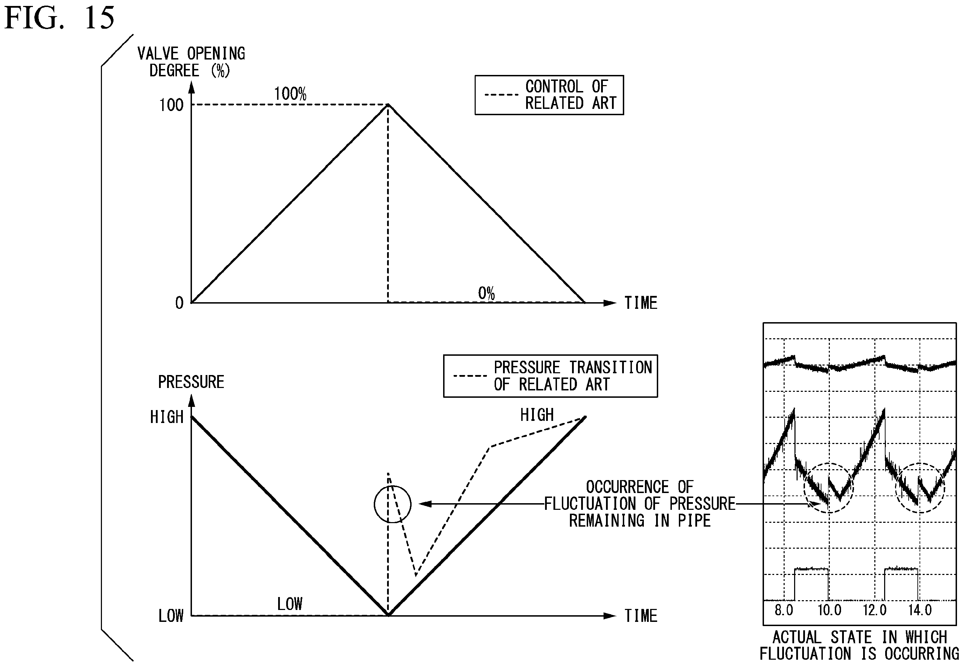

FIG. 15 is a view illustrating Example 1 of the effects of the CPAP system of the embodiment of the present invention.

An upper diagram of FIG. 15 illustrates a comparative example of the valve opening degree. As illustrated in the upper diagram of FIG. 15, in the related-art valve control, control is performed when the valve opening degree is either 0% or 100%. On the other hand, in the embodiment, the valve opening degree is controlled from 100% to 0% over time.

A lower diagram of FIG. 15 illustrates a comparative example of the pressure. As illustrated in the lower diagram of FIG. 15, in a case where the related-art valve control is performed, the pressure changes rapidly when the valve opening degree is controlled from 100% to 0%. Due to the sharp change of the pressure, the pressure remains in the tube 300 (pipe), and fluctuation due to the remaining pressure is observed.

On the other hand, in the embodiment, since the valve opening degree is controlled from 0% to 100% over time, the pressure also gradually decreases over time. In addition, in the embodiment, since the valve opening degree is controlled from 100% to 0% over time, the pressure also gradually rises over time.

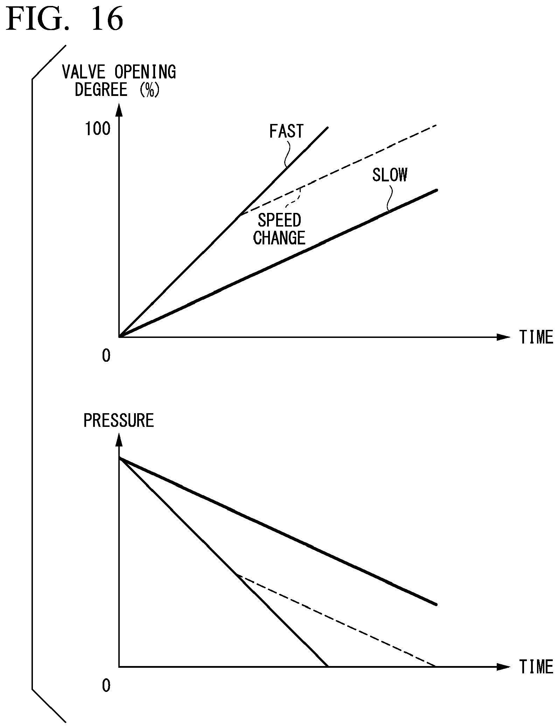

FIG. 16 is a view illustrating Example 2 of the effects of the CPAP system of the embodiment of the present invention.

In the CPAP apparatus 200 of the present embodiment, the valve opening degree can be optionally set. With such a configuration, the pressure release amount can be adjusted for each patient.

In addition, as illustrated in an upper diagram of FIG. 16, the CPAP apparatus 200 can optionally set the opening and closing speed of the valve. With such a configuration, the pressure release rate can be adjusted for each patient as illustrated in a lower diagram of FIG. 16.

In addition, as illustrated in the upper diagram of FIG. 16, the CPAP apparatus 200 can change the opening and closing speed of the valve while opening and closing the valve. With such a configuration, it is possible to suppress the swing-over of the pressure for each patient.

In the aforementioned embodiment, a case where the valve 250 is provided in the CPAP apparatus 200 has been described. However, the present invention is not limited to this example. For example, the valve 250 may be provided on the tube 300 or the mask 400.

Since the valve 250 and the pressure sensor 220 are installed at close positions by providing the CPAP apparatus 200 with the valve 250, the time from when the patient starts inhaling air until the valve 250 is closed, and the time from when the patient starts to exhale air until the valve 250 opens can be shortened as compared to a case where the valve 250 is provided at another position.

In the aforementioned embodiment, the shutter valve has been described as an example of the linear valve. However, the invention is not limited to this example. For example, as an example of the linear valve, a valve whose opening degree can be adjusted in a sliding manner or a valve whose opening degree is adjusted by sandwiching a tube-shaped tube may be used.

In the aforementioned embodiment, the servo motor has been described as an example of a valve drive mechanism. However, the present invention is not limited to this example. For example, a DC motor, an AC motor, or a stepping motor may be used as the valve drive mechanism as long as the turning angle can be controlled.

In the aforementioned embodiment, both a case where the CPAP apparatus 200 performs the processing of slowly closing the valve 250 and a case where the CPAP apparatus 200 performs the processing of slowly opening the valve 250 have been described. However, the present invention is not limited to this example. For example, the CPAP apparatus 200 may perform any one of the processing of slowly opening the valve 250 and the processing of slowly closing the valve 250.

In the aforementioned embodiment, a case where the PWM signals, which are the information indicating the rotation speed output by the motor drive unit 240, are the U-phase output voltage, the V-phase output voltage, and the W-phase output voltage has been described. However, the present invention is not limited to this example.

For example, the present invention can also be applied to a case where the PWM signals, which are the information indicating the rotation speed output by the motor drive unit 240 are a U-phase H-side output voltage, a V-phase H-side output voltage, a W-phase H-side output voltage, a U-phase L-side output voltage, a V-phase L-side output voltage, and a W-phase L-side output voltage.

A case where the PWM signals are the U-phase H-side output voltage, the V-phase H-side output voltage, the W-phase H-side output voltage, the U-phase L-side output voltage, the V-phase L-side output voltage, and the W-phase L-side output voltage will be described. In this case, FIG. 1 can be applied to an example of the CPAP system, and FIG. 4 can be applied to an example of the CPAP apparatus.

The motor drive unit 240 outputs a PWM signal as the information indicating the rotation speed. Each of the U-phase H-side output voltage, the V-phase H-side output voltage, the W-phase H-side output voltage, the U-phase L-side output voltage, the V-phase L-side output voltage, and the W-phase L-side output voltage, which are the PWM signals output by the motor drive unit 240, is supplied to the motor 230.

Specifically, the U-phase H-side output voltage and the U-phase L-side output voltage are supplied to the U-phase coil of the motor 230, the V-phase H-side output voltage and the V-phase L-side output voltage are supplied to the V-phase coil the motor 230, and the W-phase H-side output voltage and the W-phase L-side output voltage are supplied to the W-phase coil of the motor 230.

Moreover, each of the U-phase H-side output voltage, the V-phase H-side output voltage, the W-phase H-side output voltage, the U-phase L-side output voltage, the V-phase L-side output voltage, and the W-phase L-side output voltage, which are the PWM signals output by the motor drive unit 240, is output to the voltage level conversion unit 261 of the valve drive unit 260.

The voltage level conversion unit 261 steps downs the voltage level of each of the U-phase H-side output voltage, the V-phase H-side output voltage, the W-phase H-side output voltage, the U-phase L-side output voltage, the V-phase L-side output voltage, and the W-phase L-side output voltage output by the motor drive unit 240 to a voltage of 3 V, 5 V, or the like, and each of the U-phase H-side output voltage, the V-phase H-side output voltage, the W-phase H-side output voltage, the U-phase L-side output voltage, the V-phase L-side output voltage, and the W-phase L-side output voltage with the stepped-down voltage levels is output to the synthesis unit 262.

By stepping down the voltage level of each of the U-phase H-side output voltage, the V-phase H-side output voltage, the W-phase H-side output voltage, the U-phase L-side output voltage, the V-phase L-side output voltage, and the W-phase L-side output voltage, each of the U-phase H-side output voltage, the V-phase H-side output voltage, the W-phase H-side output voltage, the U-phase L-side output voltage, the V-phase L-side output voltage, and the W-phase L-side output voltage output by the motor drive unit 240 can be adjusted so as to be output to the subsequent stage.

In addition, since the low-voltage-resistant circuit can be used and configured by stepping down the voltage level of each of the U-phase H-side output voltage, the V-phase H-side output voltage, the W-phase H-side output voltage, the U-phase L-side output voltage, the V-phase L-side output voltage, and the W-phase L-side output voltage, the voltage level conversion unit 261 can be realized at low cost.

Hereinafter, as an example, a case where the voltage level of each of the U-phase H-side output voltage, the V-phase H-side output voltage, the W-phase H-side output voltage, the U-phase L-side output voltage, the V-phase L-side output voltage, and the W-phase L-side output voltage is stepped down to 5 V will continue being described.

The synthesis unit 262 acquires each of the U-phase H-side output voltage, the V-phase H-side output voltage, the W-phase H-side output voltage, the U-phase L-side output voltage, the V-phase L-side output voltage, and the W-phase L-side output voltage the voltage level output by the voltage level conversion unit 261, and synthesizes the acquired U-phase H-side output voltage, V-phase H-side output voltage, W-phase H-side output voltage, U-phase L-side output voltage, V-phase L-side output voltage, and W-phase L-side output voltage with the stepped-down voltage levels.

Specifically, the synthesis unit 262 logically synthesizes the acquired U-phase H-side output voltage, V-phase H-side output voltage, W-phase H-side output voltage, U-phase L-side output voltage, V-phase L-side output voltage, and W-phase L-side output voltage with the stepped-down voltage levels, thereby synthesizing the U-phase H-side output voltage, the V-phase H-side output voltage, the W-phase H-side output voltage, the U-phase L-side output voltage, the V-phase L-side output voltage, and the W-phase L-side output voltage with the stepped-down voltage levels.

The synthesis unit 262 outputs the synthesized signal obtained by synthesizing each of the U-phase H-side output voltage, the V-phase H-side output voltage, the W-phase H-side output voltage, the U-phase L-side output voltage, the V-phase L-side output voltage, and the W-phase L-side output voltage with the stepped-down voltage levels to the determination unit 263.

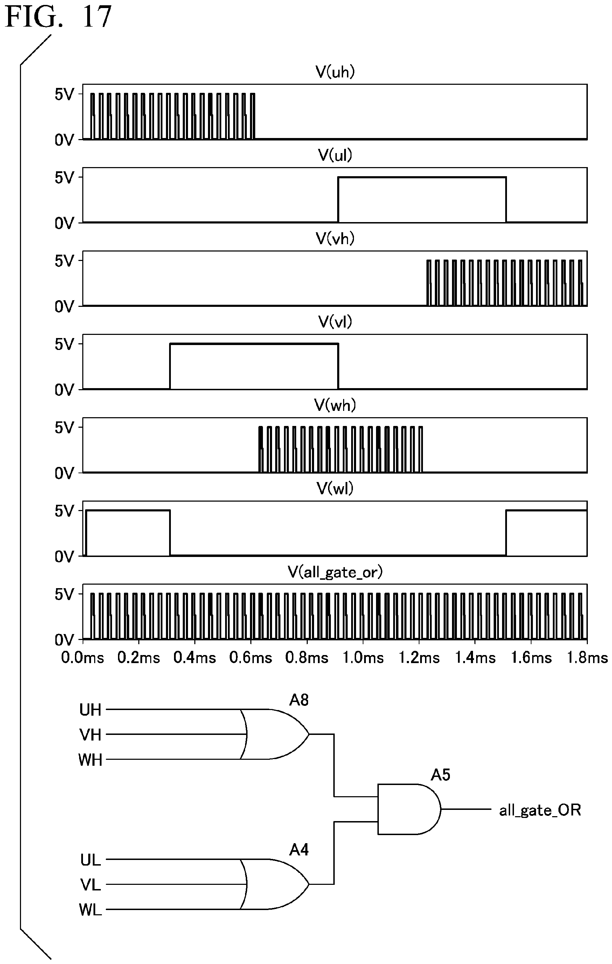

FIG. 17 is a view illustrating an example of the operation of the valve drive unit of the CPAP apparatus of the embodiment of the present invention. FIG. 17 illustrated that a first rectangular signal is obtained by synthesizing the U-phase H-side output voltage (V (uh)), the V-phase H-side output voltage (V (vh)), and the W-phase H-side output voltage (V (wh)) with the stepped-down voltage levels output by the voltage level conversion unit 261. In addition, it is illustrated that a second rectangular signal is obtained by synthesizing the U-phase L-side output voltage (V (ul)), the V-phase L-side output voltage (V (vl)), and the W-phase L-side output voltage (V (wl)). Moreover, it is illustrated that a synthesized signal is obtained by synthesizing the first rectangular signal and the second rectangular signal.

In addition, the following is illustrated in a lower part of FIG. 17.

The first rectangular signal is expressed by a logical sum of the U-phase H-side output voltage (V (uh)), the V-phase H-side output voltage (V (vh)), and the W-phase H-side output voltage (V (wh)). The second rectangular signal is expressed by a logical sum of the U-phase L-side output voltage (V (ul)), the V-phase L-side output voltage (V (vl)), and the W-phase L-side output voltage (V (wl)). The synthesized signal is expressed by a logical product of the first rectangular signal and the second rectangular signal.

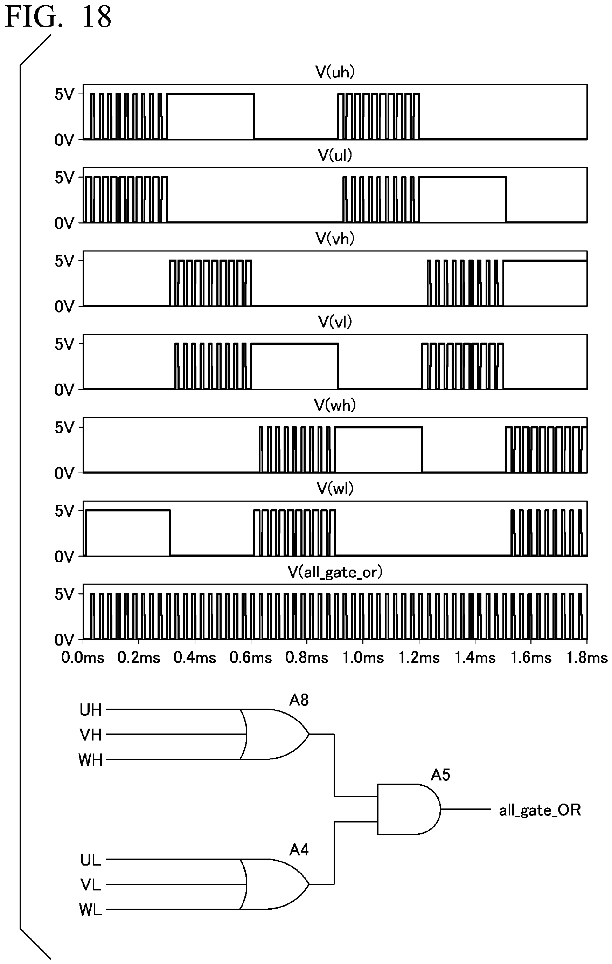

FIG. 18 is a view illustrating the operation of the valve drive unit of the CPAP apparatus of the embodiment of the present invention.

FIG. 18 illustrates that the first rectangular signal is obtained by synthesizing the U-phase H-side output voltage (V (uh)), the V-phase H-side output voltage (V (vh)), and the W-phase H-side output voltage (V (wh)) with the stepped-down voltage levels output by the voltage level conversion unit 261. In addition, it is illustrated that the second rectangular signal is obtained by synthesizing the U-phase L-side output voltage (V (ul)), the V-phase L-side output voltage (V (vl)), and the W-phase L-side output voltage (V (wl)). Moreover, it is illustrated that a synthesized signal is obtained by synthesizing the first rectangular signal and the second rectangular signal.

In addition, the following is illustrated in a lower part of FIG. 18.

The first rectangular signal is expressed by a logical sum of the U-phase H-side output voltage (V (uh)), the V-phase H-side output voltage (V (vh)), and the W-phase H-side output voltage (V (wh)). The second rectangular signal is expressed by a logical sum of the U-phase L-side output voltage (V (ul)), the V-phase L-side output voltage (V (vl)), and the W-phase L-side output voltage (V (wl)). The synthesized signal is expressed by a logical product of the first rectangular signal and the second rectangular signal.

According to the CPAP system 100 of the embodiment, the CPAP apparatus 200 includes the fan 210, the pressure sensor 220, the case 270, the motor drive unit 240, the valve 250, and the valve drive unit 260.

The fan 210 suctions and delivers air. The pressure sensor 220 measures the pressure of the air delivered by the fan 210. The case 270 has the fan 210 and the pressure sensor 220 built therein, and has the air inflow port AI that allows the air sent into the fan 210 to flow in therethrough and the air outflow port AO that allows the air delivered from the fan 210 to flow out therethrough.

The motor drive unit 240 controls the rotation speed of the motor 230 that rotates the fan 210 by a PWM signal on the basis of the pressure measured by the pressure sensor 220. The valve 250 regulates the pressure of the air supplied from the air outflow port AO via the tube 300 to the mask 400 worn on the patient PA.

The valve drive unit 260 opens and closes the valve 250 on the basis of the PWM signal. The valve drive unit 260 includes the synthesis unit 262 that synthesizes the three-phase signal for driving the motor 230 on the basis of the PWM signal, and the control unit (in the embodiment, the opening degree control unit 265 and the opening and closing speed control unit 266) that controls the closing speed at which the valve 250 is closed, and the opening degree of the valve. The valve drive unit 260 opens and closes the valve 250 on the basis of the synthesized signal obtained by synthesizing the three-phase signal by the synthesis unit 262 and the closing speed and opening degree of the valve controlled by the control unit.

In a case where the patient PA inhales air to reduce the pressure once and the fan for assisting in the inhalation is supplying air, the valve 250 can be slowly closed by controlling the closing speed and the opening degree of the valve 250. Therefore, the pressure can be gradually increased. For this reason, it is possible to improve the difficulty in breathing of the patient using CPAP.

Moreover, the control unit controls the opening speed of the valve. In a case where the pressure has been increased by the patient exhaling air, the air can be allowed to slowly flow out of the valve 250 by controlling the speed at which the valve 250 is opened. Therefore, the pressure can be gradually decreased. For this reason, it is possible to improve the difficulty in breathing of the patient using CPAP.

Moreover, the valve drive unit 260 includes the determination unit 263 that determines the rotation speed of the motor on the basis of the duty ratio of the synthesized signal. The valve drive unit 260 opens and closes the valve 250 on the basis of the result of the determination unit 263 that has determined the rotation speed of the motor. The valve drive unit 260 determines the rotation speed of the motor on the basis of the duty ratio of the synthesized signal, and can control the opening and closing speed of the valve 250 on the basis of the determination result of the rotation speed of the motor 230, thereby slowly adjusting the pressure. For this reason, it is possible to improve the difficulty in breathing of the patient using CPAP.

Moreover, the determination unit 263 determines that the motor 230 is accelerating in a case where the value obtained by converting the duty ratio of the synthesized signal into the voltage is equal to or higher than the threshold value, and determines that the motor 230 is decelerating in a case where the value obtained by converting the duty ratio of the synthesized signal into the voltage is less than the threshold value. The determination unit 263 can determine whether the motor 230 is accelerating or decelerating on the basis of the duty ratio of the synthesized signal.

Moreover, the control unit generates a control signal for opening and closing the valve on the basis of the opening degree and the opening and closing speed of the valve. With such a configuration, it is possible to make an adjustment according to the breathing of the patient PA.

### (Modification Example of Embodiment)

### (Outline of CPAP system)

FIG. 1 can be applied to an example of a CPAP system 100a of a modification example of the embodiment. However, the CPAP apparatus 200a is provided instead of the CPAP apparatus 200.

The CPAP system 100a includes the CPAP apparatus 200a, the tube 300, and the mask 400.

In the CPAP system 100a of the modification example of the embodiment, the CPAP apparatus 200a controls the opening and closing of the valve 250 on the basis of the measurement result of the flow rate of air measured by a flow rate sensor 220a.

Hereinafter, the CPAP apparatus 200a included in the CPAP system 100a will be described in detail.

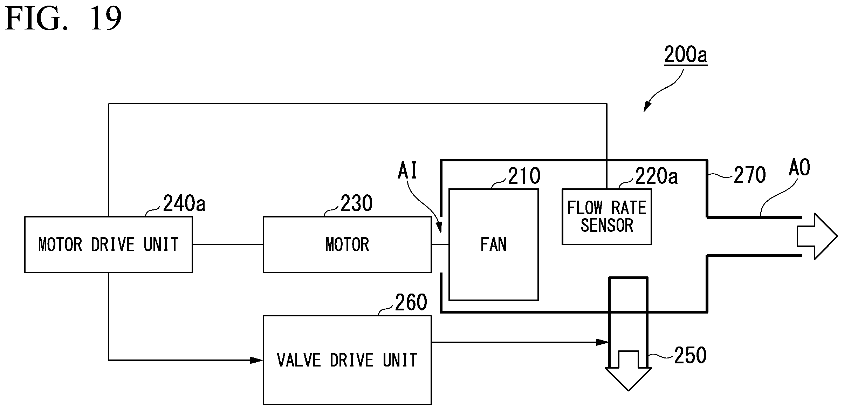

FIG. 19 is a view illustrating an example of the CPAP apparatus of the modification example of the embodiment of the present invention. As illustrated in FIG. 19, the CPAP apparatus 200a includes a fan 210, a flow rate sensor 220a, the motor 230, a motor drive unit 240a, the valve 250, the valve drive unit 260, and the case 270.

The flow rate sensor 220a is built in the case 270 and regularly measures the flow rate of the air delivered by the fan 210. The flow rate sensor 220a regularly outputs the measurement result of the flow rate of air to the motor drive unit 240a.

The motor 230 is connected to the fan 210 and rotates the fan 210 on the basis of the information indicating the rotation speed output by the motor drive unit 240a.

The motor drive unit 240a acquires the measurement result of the flow rate of air output by the flow rate sensor 220a. The motor drive unit 240a determines the rotation speed set for the motor 230 on the basis of the acquired measurement result of the flow rate of air. The motor drive unit 240a outputs information indicating the rotation speed to the motor 230 and the valve drive unit 260 on the basis of the determined rotation speed. An example of the information indicating the rotation speed output by the motor drive unit 240a is a PWM signal.

The motor drive unit 240a increases the duty ratio of the PWM signal on the basis of the acquired measurement result of the flow rate of air in a case where the measurement result of the air flow rate has increased due to the patient inhaling air. With such a configuration, since the rotation speed of the fan 210 can be increased, air can be sent into the airway of a patient who is inhaling the air, from the nose.

On the other hand, the motor drive unit 240a reduces the duty ratio on the basis of the acquired measurement result of the flow rate of air in a case where the measurement result of the air flow rate has been reduced due to the patient exhaling air. With such a configuration, since the rotation speed of the fan 210 can be reduced, the flow rate of air supplied to the mask 400 can be decrease for a patient who is exhaling air.

As an example of a characteristic diagram illustrating the response time of the CPAP system 100a of the modification example, FIG. 6 can be applied. That is, it can be seen that the rotation speed increase response time T1 is about the same, but the rotation speed reduction response time T2 is shortened. This is because, in the present modification example, in a case where the valve drive unit 260 determines that the motor 230 is decelerating due to the reduction in the flow rate, an open pulse is generated. Therefore, air leaks from the valve 250, and the pressure within the CPAP apparatus 200 decreases slowly.

### (Operation of CPAP system)

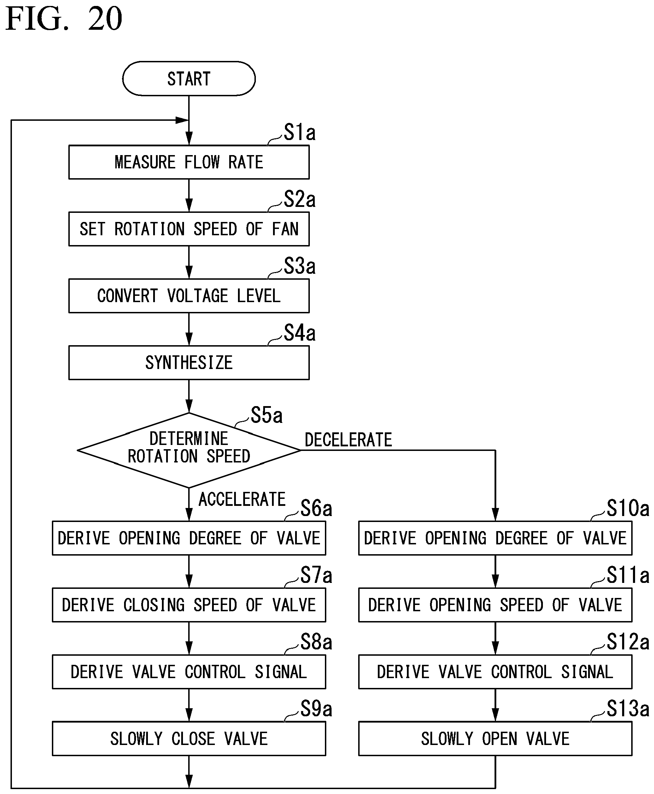

FIG. 20 is a flowchart illustrating an example of the operation of the CPAP system of the modification example of the embodiment of the present invention. FIG. 20 illustrates the operation after the CPAP system 100a is started. That is, the operation after the CPAP apparatus 200a rotates the fan 210 is illustrated.

### (Step S1a)

The flow rate sensor 220a of the CPAP apparatus 200a measures the flow rate of the air delivered by the fan 210. The flow rate sensor 220a outputs the measurement result of the flow rate of air to the motor drive unit 240a.

### (Step S2a)

The motor drive unit 240a of the CPAP apparatus 200a acquires the measurement result of the flow rate of air output by the flow rate sensor 220a, and determines the rotation speed set for the motor 230 on the basis of the acquired measurement result of the flow rate of air. The motor drive unit 240a outputs information indicating the rotation speed to the motor 230 and the valve drive unit 260 on the basis of the determined rotation speed. The motor drive unit 240a outputs a PWM signal as the information indicating the rotation speed.

The motor 230 acquires the PWM signal output by the motor drive unit 240a, and rotates the fan 210 on the basis of the acquired PWM signal.

### (Step S3a)

The PWM signal output by the motor drive unit 240a is also output to the valve drive unit 260.

The voltage level conversion unit 261 of the valve drive unit 260 acquires the U-phase output voltage, the V-phase output voltage, and the W-phase output voltage, which are the PWM signals output by the motor drive unit 240a, and steps down the voltage level of each of the acquired U-phase output voltage, V-phase output voltage, and W phase output voltage to a low voltage. The voltage level conversion unit 261 outputs each of the U-phase output voltage, the V-phase output voltage, and the W-phase output voltage with the stepped-down voltage levels to the synthesis unit 262.

### (Step S4a)

The synthesis unit 262 acquires each of the U-phase output voltage, the V-phase output voltage, and the W-phase output voltage with the stepped-down voltage levels that are output by the voltage level conversion unit 261, and synthesizes the acquired U-phase output voltage, V-phase output voltage, and W-phase output voltage with the stepped-down voltage levels. The synthesis unit 262 outputs, to the determination unit 263, a synthesized signal obtained by synthesizing each of the U-phase output voltage, the V-phase output voltage, and the W-phase output voltage with the stepped-down voltage levels.

### (Step S5a)

The determination unit 263 acquires the synthesized signal output by the synthesis unit 262, and determines the rotation speed of the motor 230 on the basis of the duty ratio of the acquired synthesized signal. In a case where the determination unit 263 determines that the motor 230 is accelerating, the determination unit 263 outputs an acceleration signal to the opening degree control unit 265 and the opening and closing speed control unit 266 and in a case where the determination unit 263 determines that the motor 230 is decelerating, the determination unit 263 outputs a deceleration signal to the opening degree control unit 265 and the opening and closing speed control unit 266.

### (Step S6a)

The opening degree setting unit 264 applies one or a plurality of threshold values to the triangular wave, and derives a time width in a case where each of the one or a plurality of threshold values is applied. The opening degree setting unit 264 derives the opening degree of the valve 250 on the basis of the derived time width. The opening degree setting unit 264 outputs the derived information indicating the opening degree of the valve 250 to the opening degree control unit 265.

### (Step S7a)

The opening and closing speed control unit 266 acquires the acceleration and deceleration signal S output by the determination unit 263. The opening and closing speed control unit 266 acquires information indicating the time for setting the opening and closing speed output by the opening and closing speed setting unit 267. The opening and closing speed control unit 266 sets the timing when the opening degree control unit 265 outputs the valve control signal for controlling the valve 250, on the basis of the triangular wave and the information indicating the time for setting the opening and closing speed. The opening and closing speed control unit 266 applies a threshold value and creates timing notification information at the timing when the triangular wave and the threshold value intersect each other. The opening and closing speed control unit 266 outputs the created timing notification information to the opening degree control unit 265.

### (Step S8a)

The opening degree control unit 265 acquires information indicating the opening degree of the valve 250 output by the opening degree setting unit 264. The opening degree control unit 265 acquires the timing notification information output by the opening and closing speed control unit 266. The opening degree control unit 265 creates the valve control signal for controlling the opening degree of the valve 250 on the basis of the acquired information indicating the opening degree of the valve 250, and outputs the created valve control signal to the valve drive motor 268 on the basis of the acquired timing notification information.

### (Step S9)

The valve drive motor 268 acquires the valve control signal output by the opening degree control unit 265, and controls the valve 250 on the basis of the acquired valve control signal. The valve 250 slowly closes on the basis of the valve control signal output by the valve drive motor 268. By slowly closing the valve 250, the amount of air flowing out from the valve 250 is gradually reduced. Therefore, it is possible to prevent the pressure from rapidly rising in a case where the fan is supplying air to assist in the inhalation.

### (Step S10a)

The opening degree setting unit 264 applies one or a plurality of threshold values to the triangular wave, and derives a time width in a case where each of the one or a plurality of threshold values is applied. The opening degree setting unit 264 derives the opening degree of the valve 250 on the basis of the derived time width of the deceleration signal. The opening degree setting unit 264 outputs the derived information indicating the opening degree of the valve 250 to the opening degree control unit 265.

### (Step S11a)

The opening and closing speed control unit 266 acquires the acceleration and deceleration signal S output by the determination unit 263. The opening and closing speed control unit 266 acquires information indicating the time for setting the opening and closing speed output by the opening and closing speed setting unit 267. The opening and closing speed control unit 266 sets the timing when the opening degree control unit 265 outputs the valve control signal for controlling the valve 250, on the basis of the triangular wave and the information indicating the time for setting the opening and closing speed. The opening and closing speed control unit 266 applies a threshold value and creates timing notification information at the timing when the triangular wave and the threshold value intersect each other. The opening and closing speed control unit 266 outputs the created timing notification information to the opening degree control unit 265.

### (Step S12a)

The opening degree control unit 265 acquires information indicating the opening degree of the valve 250 output by the opening degree setting unit 264. The opening degree control unit 265 acquires the timing notification information output by the opening and closing speed control unit 266. The opening degree control unit 265 creates the valve control signal for controlling the opening degree of the valve 250 on the basis of the acquired information indicating the opening degree of the valve 250, and outputs the created valve control signal to the valve drive motor 268 on the basis of the acquired timing notification information.

### (Step S13a)

The valve drive motor 268 acquires the valve control signal output by the opening degree control unit 265, and controls the valve 250 on the basis of the acquired valve control signal. The valve 250 opens slowly on the basis of the valve control signal output by the valve drive motor 268. By slowly opening the valve 250, air gradually flows out from the valve 250.

In the aforementioned modification example, a case where the CPAP apparatus 200a is provided with the valve 250 has been described. However, the present invention is not limited to this example.

For example, the valve 250 may be provided on the tube 300 or the mask 400. Since the valve 250 and the pressure sensor 220 are installed at close positions by providing the CPAP apparatus 200a with the valve 250, the time from when the patient starts to inhale air until the valve 250 is closed, and the time from when the patient starts to exhale air until the valve 250 opens can be shortened as compared to a case where the valve 250 is provided at another position.

In the aforementioned modification example, a case where the PWM signals, which are the information indicating the rotation speed output by the motor drive unit 240a, are the U-phase output voltage, the V-phase output voltage, and the W-phase output voltage has been described. However, the present invention is not limited to this example.

For example, the present invention can also be applied to a case where the PWM signals, which are the information indicating the rotation speed output by the motor drive unit 240a are a U-phase H-side output voltage, a V-phase H-side output voltage, a W-phase H-side output voltage, a U-phase L-side output voltage, a V-phase L-side output voltage, and a W-phase L-side output voltage.

A case where the PWM signals are the U-phase H-side output voltage, the V-phase H-side output voltage, the W-phase H-side output voltage, the U-phase L-side output voltage, the V-phase L-side output voltage, and the W-phase L-side output voltage will be described. In this case, FIG. 1 can be applied to an example of the CPAP system, and FIG. 19 can be applied to an example of the CPAP apparatus.

The motor drive unit 240a outputs a PWM signal as the information indicating the rotation speed. Each of the U-phase H-side output voltage, the V-phase H-side output voltage, the W-phase H-side output voltage, the U-phase L-side output voltage, the V-phase L-side output voltage, and the W-phase L-side output voltage, which are the PWM signals output by the motor drive unit 240a, is supplied to the motor 230.

Specifically, the U-phase H-side output voltage and the U-phase L-side output voltage are supplied to the U-phase coil of the motor 230, the V-phase H-side output voltage and the V-phase L-side output voltage are supplied to the V-phase coil the motor 230, and the W-phase H-side output voltage and the W-phase L-side output voltage are supplied to the W-phase coil of the motor 230.

Moreover, each of the U-phase H-side output voltage, the V-phase H-side output voltage, the W-phase H-side output voltage, the U-phase L-side output voltage, the V-phase L-side output voltage, and the W-phase L-side output voltage, which are the PWM signals output by the motor drive unit 240a, is output to the voltage level conversion unit 261 of the valve drive unit 260.

The voltage level conversion unit 261 steps down the voltage level of each of the U-phase H-side output voltage, the V-phase H-side output voltage, the W-phase H-side output voltage, and the U-phase L-side output voltage, the V-phase L-side output voltage, and the W-phase L-side output voltage output by the motor drive unit 240a to a voltage such as 3 V or 5 V, and outputs each of the U-phase H-side output voltage, the V-phase H-side output voltage, the W-phase H-side output voltage, the U-phase L-side output voltage, the V-phase L-side output voltage, and the W-phase L-side output voltage with the steppe-down voltage levels to the synthesis unit 262.

By stepping down the voltage level of each of the U-phase H-side output voltage, the V-phase H-side output voltage, the W-phase H-side output voltage, the U-phase L-side output voltage, the V-phase L-side output voltage, and the W-phase L-side output voltage, each of the U-phase H-side output voltage, the V-phase H-side output voltage, the W-phase H-side output voltage, the U-phase L-side output voltage, the V-phase L-side output voltage, and the W-phase L-side output voltage output by the motor drive unit 240a can be adjusted so as to be output to the subsequent stage.

In addition, since the low-voltage-resistant circuit can be used and configured by stepping down the voltage level of each of the U-phase H-side output voltage, the V-phase H-side output voltage, the W-phase H-side output voltage, the U-phase L-side output voltage, the V-phase L-side output voltage, and the W-phase L-side output voltage, the voltage level conversion unit 261 can be realized at low cost.

Hereinafter, as an example, a case where the voltage level of each of the U-phase H-side output voltage, the V-phase H-side output voltage, the W-phase H-side output voltage, the U-phase L-side output voltage, the V-phase L-side output voltage, and the W-phase L-side output voltage is stepped down to 5 V will continue being described.

The synthesis unit 262 acquires each of the U-phase H-side output voltage, the V-phase H-side output voltage, the W-phase H-side output voltage, the U-phase L-side output voltage, the V-phase L-side output voltage, and the W-phase L-side output voltage the voltage level output by the voltage level conversion unit 261, and synthesizes the acquired U-phase H-side output voltage, V-phase H-side output voltage, W-phase H-side output voltage, U-phase L-side output voltage, V-phase L-side output voltage, and W-phase L-side output voltage with the stepped-down voltage levels.

Specifically, the synthesis unit 262 logically synthesizes the acquired U-phase H-side output voltage, V-phase H-side output voltage, W-phase H-side output voltage, U-phase L-side output voltage, V-phase L-side output voltage, and W-phase L-side output voltage with the stepped-down voltage levels, thereby synthesizing the U-phase H-side output voltage, the V-phase H-side output voltage, the W-phase H-side output voltage, the U-phase L-side output voltage, the V-phase L-side output voltage, and the W-phase L-side output voltage with the stepped-down voltage levels.

The synthesis unit 262 outputs the synthesized signal obtained by synthesizing each of the U-phase H-side output voltage, the V-phase H-side output voltage, the W-phase H-side output voltage, the U-phase L-side output voltage, the V-phase L-side output voltage, and the W-phase L-side output voltage with the stepped-down voltage levels to the determination unit 263.

FIGS. 19 and 20 can be applied to the views illustrating an example of the operation of the valve drive unit 260 of the CPAP apparatus 200a of the modification example of the embodiment of the present invention.

According to the CPAP system 100a of the modification example, the CPAP apparatus 200a includes the fan 210, the flow rate sensor 220a, the case 270, the motor drive unit 240a, the valve 250, and the valve drive unit 260.

The fan 210 suctions and delivers air. The flow rate sensor 220a measures the flow rate of the air delivered by the fan 210. The case 270 has the fan 210 and the flow rate sensor 220a built therein, and has the air inflow port AI that allows the air sent into the fan 210 to flow in therethrough and the air outflow port AO that allows the air delivered from the fan 210 to flow out therethrough.

The motor drive unit 240a controls the rotation speed of the motor 230 that rotates the fan 210 by a PWM signal on the basis of the flow rate measured by the flow rate sensor 220a. The valve 250 regulates the pressure of the air supplied from the air outflow port AO via the tube 300 to the mask 400 worn on the patient PA.

The valve drive unit 260 opens and closes the valve 250 on the basis of the PWM signal. The valve drive unit 260 includes the synthesis unit 262 that synthesizes the three-phase signal for driving the motor 230 on the basis of the PWM signal, and the control unit (in the embodiment, the opening degree control unit 265 and the opening and closing speed control unit 266) that controls the closing speed at which the valve 250 is closed, and the opening degree of the valve. The valve drive unit 260 opens and closes the valve 250 on the basis of the synthesized signal obtained by synthesizing the three-phase signal by the synthesis unit 262 and the closing speed and opening degree of the valve controlled by the control unit.

In a case where the patient PA has increased the flow rate by inhaling air, the valve 250 can be slowly closed by controlling the closing speed of the valve 250. Therefore, the pressure can be gradually increased. For this reason, it is possible to improve the difficulty in breathing of the patient using CPAP.

### (Valve Modification Example)

As mentioned earlier, the valve of the present embodiment is not limited to the shutter valve illustrated in FIG. 13, and the pressure or flow rate of the air supplied from the air outflow port AO via the tube 300 to the mask 400 is not limited to a specific configuration as long as the pressure or flow rate can be regulated.

For example, a valve using a movable plate may be used. A modification example of the valve in this case will be described in detail below.

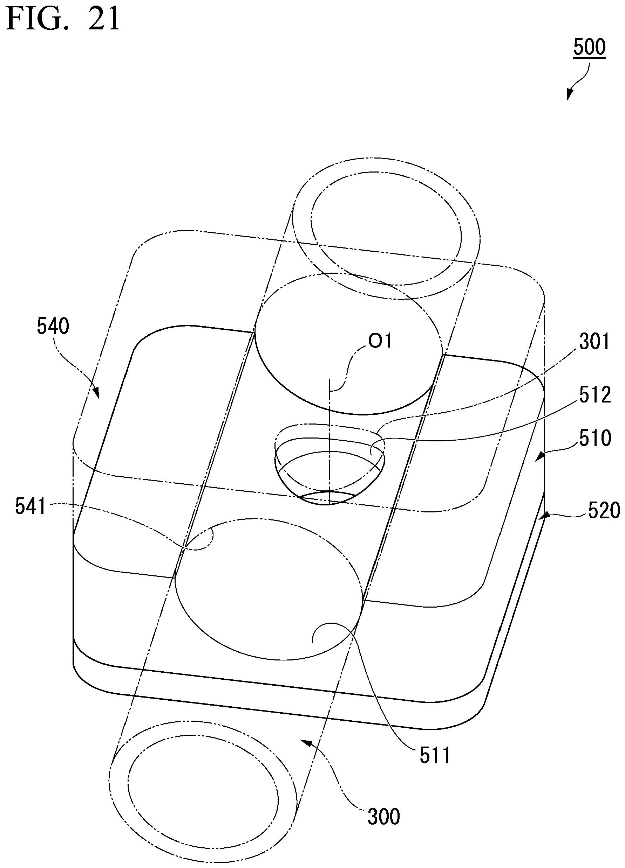

As illustrated in FIG. 21, the valve 500 adjusts the pressure or flow rate of the air supplied to the mask 400 worn on the patient PA (refer to FIG. 1) via the tube 300, and is integrally combined with the tube 300 and used.

In this case, the valve 500 may be used in a state of being combined with a portion of the tube 300 located between the case 270 and the mask 400 illustrated in FIG. 1, or may be used in a state of being combined with the tube 300 within the case 270.

As illustrated in FIGS. 21 to 23, the valve 500 includes a support plate (a first fixing member according to the present invention) 510 disposed on an outer peripheral side of the tube 300 and combined with the tube 300, a base plate (a second fixing member according to the present invention) 520 combined with the support plate 510, and a rotary plate (a movable plate according to the present invention) 530 disposed between the support plate 510 and the base plate 520 and configured to be movable relative to the support plate 510 and the base plate 520.

As illustrated in FIG. 21, the tube 300 has an exhaust hole (first air discharge hole according to the present invention) 301 that is formed so as to penetrate the tube 300 in the radial direction and allows the inside and the outside of the tube 300 to communicate with each other. The exhaust hole 301 is open to an outer peripheral surface of the tube 300 so as to face the support plate 510. Accordingly, the air delivered into the tube 300 through the air outflow port AO by the fan 210 illustrated in FIG. 4 can be discharged to the outside of the tube 300 through the exhaust hole 301.

In addition, in the present modification example, the shape of the exhaust hole 301 is circular as illustrated in FIG. 21. Also, an axis extending in the radial direction of the tube 300 while passing through the center of the exhaust hole 301 is referred to as a first axis O1. Moreover, a direction along the first axis O1 is referred to as an up-down direction, and the direction of the up-down direction from the support plate 510 toward the tube 300 is referred to as an upward direction, and the opposite direction is referred to as a downward direction.

### (Support Plate)

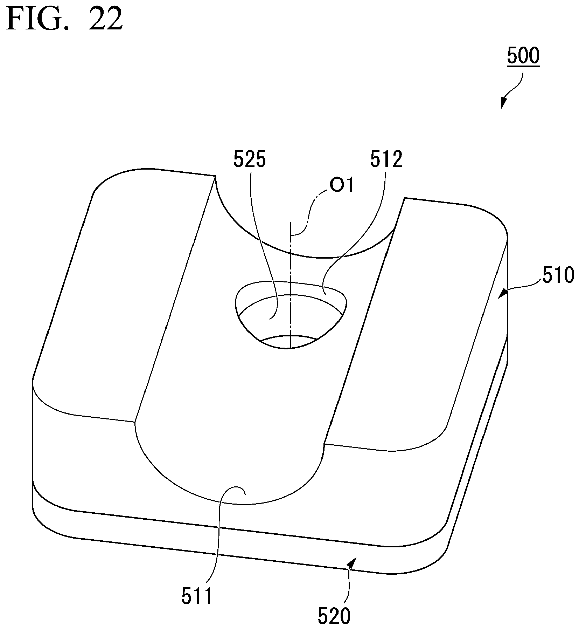

As illustrated in FIGS. 22 to 24, the support plate 510 is formed in a quadrangular shape in plan view having a predetermined thickness. However, the outer shape of the support plate 510 is not limited to this case, and may be appropriately changed.

A curved groove 511 that curves according to the curvature of an outer peripheral surface of the tube 300 and extends along the tube 300 is formed on an upper surface of the support plate 510 located on the tube 300 side. The support plate 510 is integrally combined with the tube 300 via a known fastening member or the like (not illustrated) in a state where the curved groove 511 is brought into surface contact with the outer peripheral surface of the tube 300.

In addition, as illustrated in FIG. 21, the valve 500 further includes an auxiliary plate 540, and the support plate 510 and the auxiliary plate 540 may be integrally combined via a known fastening member (not illustrated) so as to sandwich the tube 300 therebetween. A curved groove 541 similar to the curved groove 511 is formed on a lower surface of the auxiliary plate 540 located on the tube 300 side.

However, the auxiliary plate 540 is not essential and may not be provided.

As illustrated in FIGS. 22 to 24, the support plate 510 is formed with a communication hole 512 that penetrates the support plate 510 in the up-down direction and that communicates with the exhaust hole 301 formed in the tube 300. The communication hole 512 is formed, for example, in a circular shape in plan view, and has the same opening size as the exhaust hole 301.

The communication hole 512 is formed at a position eccentric in an extending direction of the tube 300 with respect to a second axis 02 (a rotation axis according to the present invention) that penetrates the center of the support plate 510 in the up-down direction. In addition, in the present modification example, in a plan view viewed from the direction of the second axis 02, a direction intersecting the second axis 02 is referred to as a radial direction and a direction around the second axis 02 is referred to as a circumferential direction.

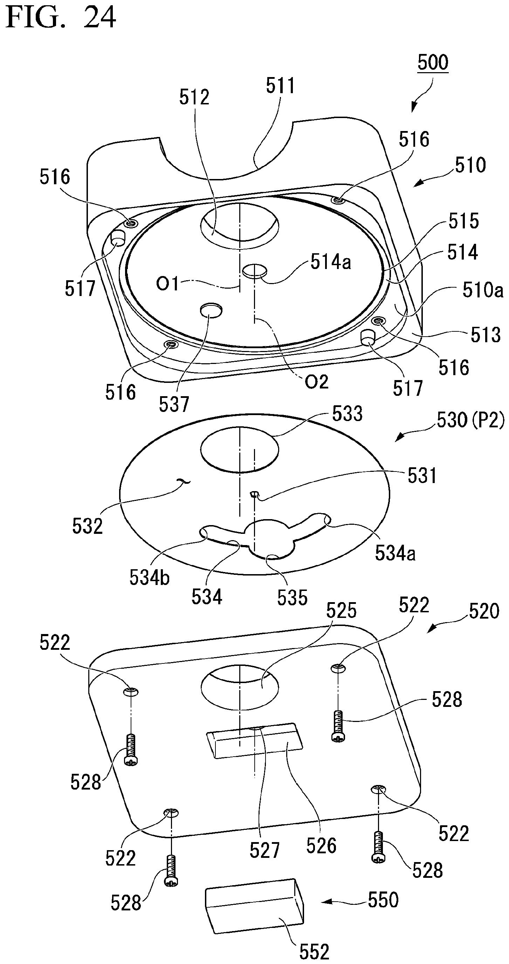

As illustrated in FIG. 24, an annular protruding wall 513 is formed on a lower surface 510a of the support plate 510 so as to protrude downward along an outer peripheral edge of the support plate 510. Moreover, an accommodation recess 514 having a circular shape in plan view which is recessed upward is formed at a central portion of the lower surface 510a of the support plate 510.

Since the present invention is configured in this way, the lower surface 510a of the support plate 510 is recessed from the protruding wall 513 by one step, and the accommodation recess 514 is formed to be recessed from the lower surface 510a by one step.

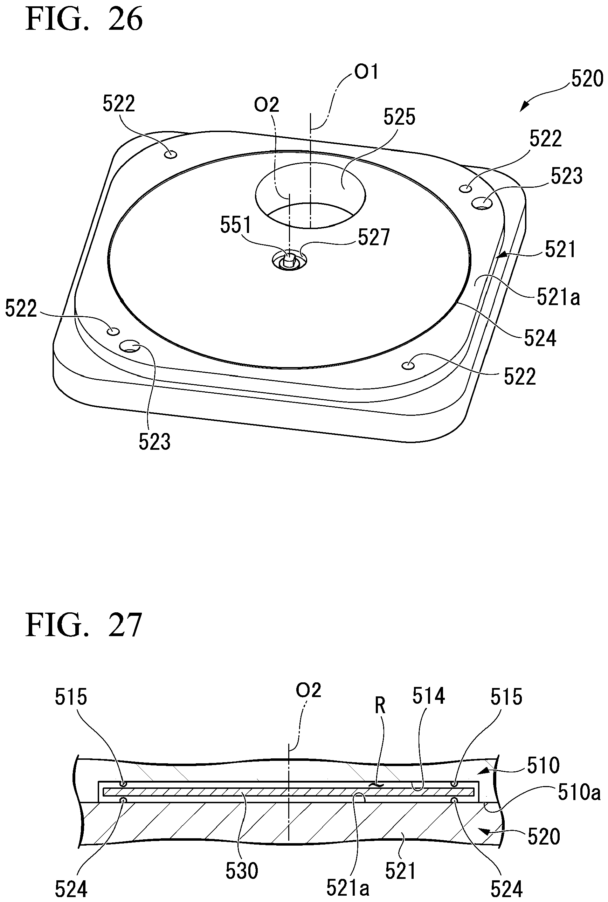

The accommodation recess 514 has a diameter slightly larger than the diameter of the rotary plate 530, and is formed in a circular shape in plan view with the second axis 02 as the center. An annular first guide protrusion 515 slightly protruding downward is formed on a top wall of the accommodation recess 514 coaxially with the second axis 02. A first guide protrusion 515 is formed with a diameter slightly smaller than the diameter of the rotary plate 530, and is formed so as to bulge downward in a hemispherical shape in a longitudinal sectional view (refer to FIG. 27).

Moreover, screw holes 516 are formed in the lower surface 510a in the vicinity of the four corner portions (four corners) of the support plate 510, respectively. In addition, among the four corner portions of the support plate 510, positioning protrusions 517 protruding downward are formed on the lower surface 510a in the vicinity of a pair of corner portions that face each other in the radial direction with the second axis 02 interposed therebetween. In addition, the positioning protrusions 517 are formed adjacent to the screw holes 516.

### (Base Plate)

As illustrated in FIGS. 22 to 24, the base plate 520 is combined from below with the support plate 510 configured as described above.

The base plate 520 is formed in a quadrangular shape in plan view having a predetermined thickness corresponding to the outer shape of the support plate 510. In addition, the outer size of the base plate 520 is the same as the outer size of the support plate 510.

A bulging part 521 that bulges upward is formed at a central portion of an upper surface of the base plate 520. The bulging part 521 is shaped to fit inside the annular protruding wall 513 of the support plate 510, and protrudes upward with a protruding amount equal to the protruding amount of the protruding wall 513. In addition, an upper surface 521a of the bulging part 521 is a flat surface that can contact the lower surface 510a of the support plate 510 from below.

Accordingly, when the base plate 520 is combined with the support plate 510 from below, the accommodation recess 514 formed in the support plate 510 can be closed from below by the bulging part 521, and the inside of the accommodation recess 514 can be made to function as an accommodation space R (refer to FIG. 27).

The base plate 520 is formed with four through holes 522 that penetrate the base plate 520 in the up-down direction. The four through holes 522 are formed so as to face the respective screw holes 516 formed in the support plate 510 in the up-down direction, and is open to the upper surface 521a of the bulging part 521.

Moreover, a pair of positioning holes 523 is formed on the upper surface 521a of the bulging part 521 so as to be depressed downward. The pair of positioning holes 523 is formed so as to face the respective positioning protrusions 517 formed on the support plate 510 in the up-down direction, and the positioning protrusions 517 can be inserted therein.

On the upper surface 521a of the bulging part 521, an annular second guide protrusion 524 slightly protruding upward is formed coaxially with the second axis 02. The second guide protrusion 524 is formed to have the same diameter as the diameter of the first guide protrusion 515, and is formed so as to bulge upward in a hemispherical shape in a longitudinal sectional view (refer to FIG. 27). For that reason, the second guide protrusion 524 is disposed so as to face the first guide protrusion 515 in the up-down direction.

Moreover, the base plate 520 is formed with an air discharge hole (a second air discharge hole according to the present invention) 525 disposed to penetrate the base plate 520 in the up-down direction and to face the communication hole 512 formed in the support plate 510 in the up-down direction.

The air discharge hole 525 is disposed coaxially with the first axis O1 and is formed in a circular shape in a plan view. Specifically, the air discharge hole 525 is formed to have the same diameter as the communication hole 512. The air discharge hole 525 allows the inside and the outside of the above-described accommodation space R to communicate with each other.

Moreover, a storage recess 526 for storing a drive motor 550, to be described below is formed at a central portion of the lower surface 510a of the base plate 520 so as to be recessed upward.

In the illustrated example, the storage recess 526 is formed in a rectangular shape in plan view, and has a depth that allows the drive motor 550 to be completely stored therein. However, the shape of the storage recess 526 is not limited to the rectangular shape in plan view, and may be formed to correspond to the shape of the drive motor 550.

Also, a shaft hole 527 is formed at the central portion of the base plate 520 so as to penetrate the base plate 520 in the up-down direction, be open to the upper surface 521a of the bulging part 521, and communicate with the storage recess 526. The shaft hole 527 is disposed coaxially with the second axis 02, and is formed in a circular shape in plan view.

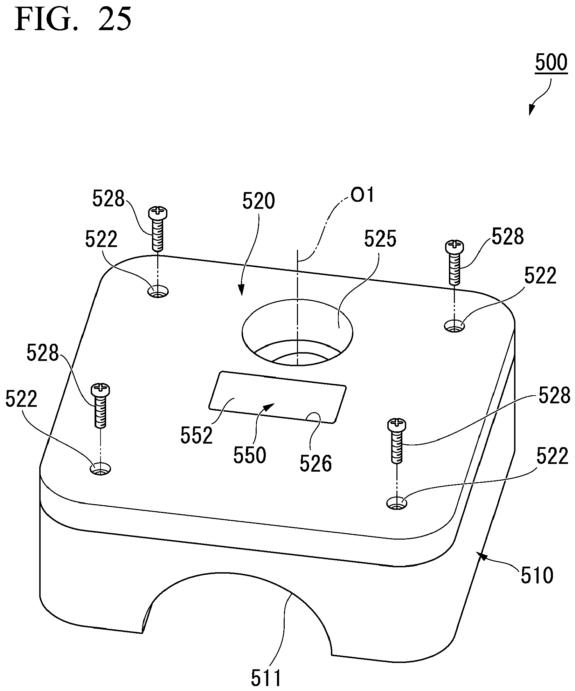

The base plate 520 configured as described above can be superimposed on the support plate 510 from below while inserting the positioning protrusion 517 into the positioning hole 523, and then, as illustrated in FIGS. 24 and 25, is integrally combined with the support plate 510 by screwing a coupling screw 528 into the screw hole 516 through the through hole 522.

### (Drive Motor)

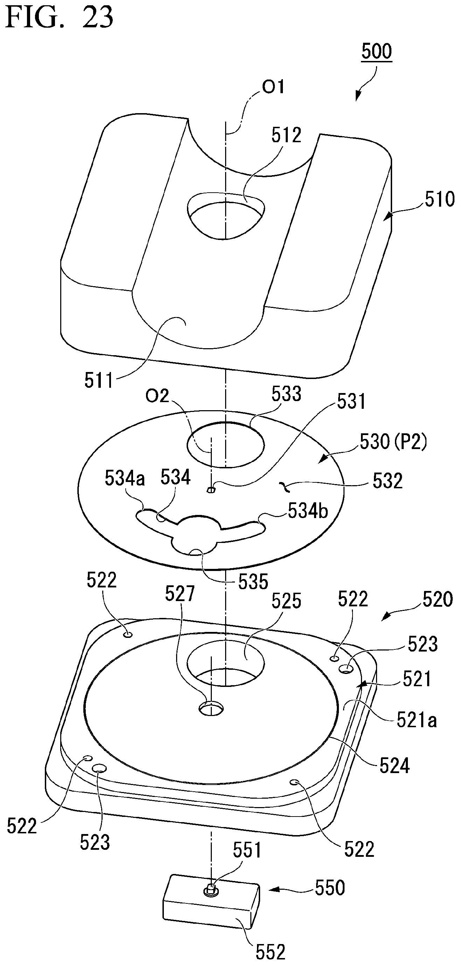

As illustrated in FIG. 23, the drive motor 550 that functions as the valve drive motor 268 illustrated in FIG. 7 is attached to the base plate 520 using the above-described storage recess 526.

The drive motor 550 is, for example, a geared motor, and includes a stepping motor (not illustrated), a speed reducer having an output shaft 551, and a motor case 552 having the stepping motor and the speed reducer built therein.

The motor case 552 is formed in a rectangular parallelepiped shape, can be incorporated into the storage recess 526 from below, and is completely stored in the storage recess 526.

The stepping motor includes a motor shaft (not illustrated) that makes one rotation in a predetermined number of steps. The speed reducer has the output shaft 551 that rotates with the rotation of the motor shaft. The output shaft 551 is inserted into the shaft hole 527 from below and is disposed coaxially with the second axis 02. Accordingly, the output shaft 551 is rotatable around the second axis 02 with the rotation of the motor shaft. In addition, the output shaft 551 rotates around the second axis 02 with the rotation of the motor shaft while decelerating at a predetermined reduction ratio by the speed reducer.

As illustrated in FIG. 26, an upper end of the output shaft 551 slightly protrudes above the second guide protrusion 524 of the base plate 520. In addition, as illustrated in FIG. 24, an escape hole 514a for avoiding contact with the upper end of the output shaft 551 is formed at a central portion of a top wall of the accommodation recess 514 of the support plate 510 so as to be recessed upward. In addition, the upper end of the output shaft 551 is formed in a non-circular shape in plan view by a cut surface or the like.

### (Rotary Plate)

As illustrated in FIGS. 23 and 27, the rotary plate 530 is a disc plate having a predetermined thickness, and is disposed in the accommodation space R so as to be rotatable around the second axis 02. The rotary plate 530 is formed such that its diameter is larger than the diameters of the first guide protrusion 515 and the second guide protrusion 524 and smaller than the inner diameter of the accommodation recess 514. For that reason, as illustrated in FIG. 27, the rotary plate 530 is disposed within the accommodation space R in a state where which an outer peripheral edge is rotationally guided by the first guide protrusion 515 and the second guide protrusion 524 with a slight gap from the up-down direction.

In addition, FIG. 27 is a view mainly illustrating a relationship between the rotary plate 530, the first guide protrusion 515, and the second guide protrusion 524, and illustration of the shaft hole 527, the output shaft 551, and the like are omitted.

Accordingly, the rotary plate 530 is restricted to some extent in the up-down direction and is rotatable around the second axis 02 with less rattling. Moreover, the rotary plate 530 does not make surface contact with the accommodation recess 514 of the support plate 510 and the bulging part 521 of the base plate 520, and is disposed with a slight gap between the first guide protrusion 515 and the second guide protrusion 524. Accordingly, even if the rotary plate 530 rotates so as to swing in the up-down direction due to the accuracy of each part and the assembly accuracy, the rotary plate 530 only contacts the first guide protrusion 515 and the second guide protrusion 524. Thus, the frictional resistance is suppressed. For that reason, the rotary plate 530 can be smoothly rotated with less resistance.

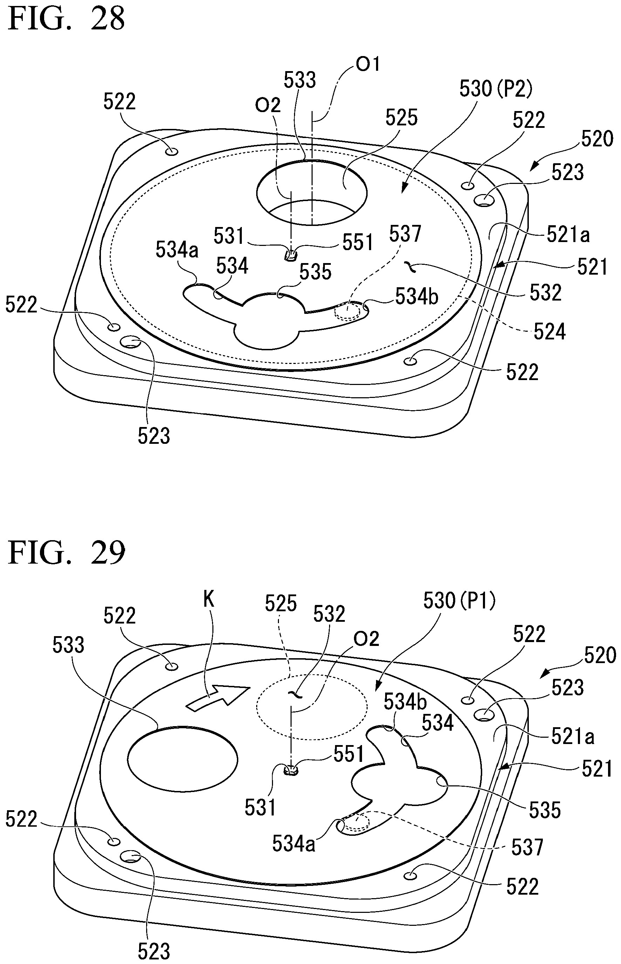

As illustrated in FIGS. 23 and 28, a coupling hole 531 that can be coupled to the upper end of the output shaft 551 is formed at the central portion of the rotary plate 530. The coupling hole 531 is formed in a non-circular shape in plan view corresponding to the shape of the upper end of the output shaft 551, and can be coupled to the upper end of the output shaft 551 by, for example, fitting. In addition, in addition to the above-described fitting or the like, it is preferable to further tightly couple the upper end of the output shaft 551 and the coupling hole 531 to each other by using an adhesive or the like to suppress occurrence of play or the like between them.

Therefore, the rotary plate 530 is configured to be rotatable about the second axis 02 by the drive of the drive motor 550.

The rotary plate 530 is disposed so as to be capable of closing the air discharge hole 525 formed in the base plate 520 from above by using a closing region 532, and has an air communication hole 533 that allows the inside of the air discharge hole 525 and the inside of the accommodation space R to communicate with each other. The air communication hole 533 is formed so as to penetrate the rotary plate 530 in the up-down direction, and is also formed in a circular shape in plan view with the same diameter as the air discharge hole 525.

Also, the rotary plate 530 is reciprocally rotated around the second axis 02 between a fully closed position P1 illustrated in FIG. 29 where the air discharge hole 525 is fully closed by using the closing region 532 and a fully open position P2 illustrated in FIG. 28 where the air discharge hole 525 is fully opened through the air communication hole 533, by the driving of the drive motor 550.

Moreover, as illustrated in FIGS. 23 and 28, the rotary plate 530 is formed with a guide groove 534 that extends in the circumferential direction and is arcuate in a plan view so as to penetrate the rotary plate 530 in the up-down direction.

In the illustrated example, the guide groove 534 is disposed so as to be located on a side opposite to the air communication hole 533 with the second axis 02 interposed therebetween, and is formed so as to extend in the circumferential direction within an angle range of about 90 degrees around the second axis 02. However, the circumferential length of the guide groove 534 is not limited to this case, and may be appropriately changed.

In addition, an expansion groove 535 is formed at a central portion of the guide groove 534 in the circumferential direction so as to bulge in an arc shape radially inward and radially outward. The expansion groove 535 plays a role of adjusting the opening area such that the opening area by the expansion groove 535 and the guide groove 534 becomes equal to the opening area of the air communication hole 533. Therefore, the shape, position, number, and the like of the expansion groove 535 may be appropriately changed according to the shape, size, and the like of the guide groove 534.

In this way, since the opening area of the expansion groove 535 and the guide groove 534 and the opening area of the air communication hole 533 are set to be equal to each other, it is possible to suppress rotational shaking of the rotary plate 530 and the like and it is possible to rotate the rotary plate 530 smoothly and stably.

In addition, one peripheral end of the peripheral ends of the guide groove 534 located in the clockwise direction (hereinafter, simply referred to as the clockwise direction) with respect to the expansion groove 535 in the plan view when the base plate 520 is viewed from above is referred to as a first peripheral end 534a, and the other peripheral end thereof located in the counterclockwise direction (hereinafter, simply referred to as the counterclockwise direction) with respect to the expansion groove 535 is referred to as a second peripheral end 534b.

Meanwhile, a guide projection 537 to be inserted into the guide groove 534 is formed on at least any one of the support plate 510 and the base plate 520 so as to correspond to the above-described guide groove 534.

In the present modification example, as illustrated in FIG. 24, the guide projection 537 is formed so as to extend downward from an upper wall surface of the accommodation recess 514 of the support plate 510, and is inserted into the guide groove 534 from above as illustrated in FIG. 28. Accordingly, the guide projection 537 is relatively movable along the guide groove 534 with the rotation of the rotary plate 530.

However, the guide projection 537 is not limited to the case of being formed on the support plate 510 side, and may be formed on the base plate 520 side. In this case, for example, it is possible to form a guide projection 537 so as to extend upward from the upper surface 521a of the bulging part 521 of the base plate 520 and insert the guide projection 537 into the guide groove 534 from below.

As illustrated in FIG. 29, the guide projection 537 is disposed at a position close to the first peripheral end 534a in the guide groove 534 when the rotary plate 530 is located at the fully closed position P1, and is provided in a state where the positional relationship with the guide groove 534 is adjusted.

For that reason, in the present modification example, the rotary plate 530 moves toward the fully open position P2 illustrated in FIG. 28 by rotating around the second axis 02 in the clockwise direction as indicated by an arrow K from the fully closed position P1 illustrated in FIG. 29, and on the contrary, moves toward the fully closed position P1 by rotating around the second axis 02 in the counterclockwise direction from the fully open position P2.

In addition, as illustrated in FIG. 28, the guide projection 537 is disposed at a position close to the second peripheral end 534b in the guide groove 534 in a case where the rotary plate 530 is located at the fully open position P2.

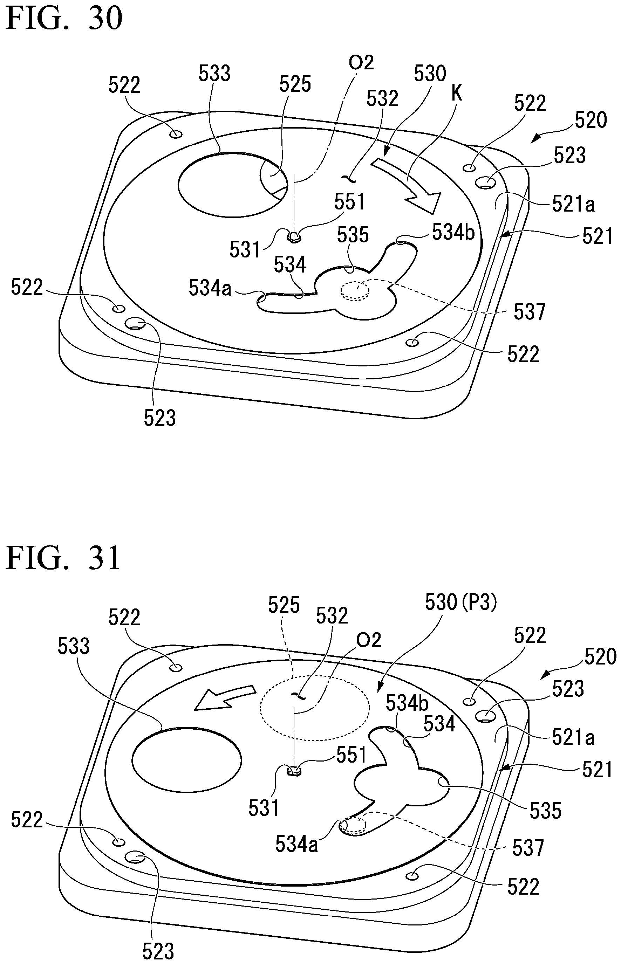

Moreover, as the rotary plate 530 rotates from the fully closed position P1 toward the fully open position P2, the air communication hole 533 moves so as to cross the air discharge hole 525 as illustrated in FIG. 30. Thus, it is possible to gradually increase the opening degree of the air discharge hole 525 through the air communication hole 533.

In particular, the guide projection 537 is brought into non-contact with the first peripheral end 534a and the second peripheral end 534b in the guide groove 534 even in a case where the rotary plate 530 is located at the fully closed position P1 or the fully open position P2. For that reason, in the stage after the CPAP apparatus 200 is started, the opening and closing operation of the valve 500 can be performed without bringing the guide projection 537 into contact with the first peripheral end 534a and the second peripheral end 534b.

In contrast, in a case where the CPAP apparatus 200 is started, the rotary plate 530 is rotated until the guide projection 537 is brought into contact with the first peripheral end 534a, so that it is possible to position the rotary plate 530 at the fully closed position P1 without utilizing a detection sensor or the like. This point will be described below again.

### (Operation of Valve)

Next, the operation of the valve 500 configured as described above will be described.

In this case, the drive motor 550 functioning as the valve drive motor acquires the valve control signal output from the opening degree control unit 265 illustrated in FIG. 7 and controls the valve 500 on the basis of the acquired valve control signal.

Specifically, the drive motor 550 rotates the output shaft 551 via the motor shaft in a predetermined number of steps. Accordingly, since the rotary plate 530 can be reciprocally rotated around the second axis 02 between the fully closed position P1 illustrated in FIG. 29 and the fully open position P2 illustrated in FIG. 28, air can be discharged from the inside of the tube 300 through the exhaust hole 301, the accommodation space R, the air communication hole 533, and the air discharge hole 525 to the outside if necessary. In addition, on the contrary, the discharge of air from the tube 300 can be suppressed.

In this way, the opening and closing of the valve 500 can be performed with a simple configuration in which the rotary plate 530 having the air communication hole 533 formed therein is moved.

In particular, since the valve 500 is disposed not on the inside of the tube 300 but on the outer peripheral side of the tube 300 as illustrated in FIG. 21, the valve 500 can be designed separately and independently from the tube 300 and can be easily downsized. Moreover, even if the rotary plate 530 is brought into an inoperative state for some reason, the rotary plate 530 does not close the inside of the tube 300. Thus, there is no concern of blocking the flow itself of the air to be supplied to the patient PA. Moreover, since the valve 500 is disposed on the outer peripheral side of the tube 300, maintenance, replacement, and the like can be easily performed, and serviceability can be improved.

In addition to this, since the rotary plate 530, unlike a so-called shutter valve having a plurality of blade members and the like, is formed in a single plate shape, the rotary plate 530 can have a certain stiffness (mechanical strength). For that reason, inconveniences such as deformation of the rotary plate 530 are less likely to occur due to the influence of pressure of air and the like, and stable opening and closing operation of the valve 500 can be performed over a long period of time.

Moreover, when the rotary plate 530 is moved from the fully closed position P1 to the fully open position P2, as illustrated in FIG. 30, the opening degree of the air discharge hole 525 can be linearly increased corresponding to the movement amount of the rotary plate 530. In addition, on the contrary, when the rotary plate 530 is moved from the fully open position P2 to the fully closed position P1, the opening degree of the air discharge hole 525 can be linearly decreased corresponding to the movement amount of the rotary plate 530. In this way, the opening degree of the valve 500 can also be linearly and finely controlled.

Moreover, as illustrated in FIGS. 28 and 29, the guide projection 537 is brought into non-contact with the first peripheral end 534a and the second peripheral end 534b of the guide groove 534 even in a case where or not the rotary plate 530 is located at the fully closed position P1 or the fully open position P2. For that reason, at the time of the opening and closing operation of the valve 500, it is possible to prevent generation of a collision sound or the like resulting from the contact between the guide projection 537 and the first peripheral end 534a and the second peripheral end 534b. Therefore, discomfort is less likely to be given to the patient PA.

Moreover, due to the contact between the guide projection 537 and the first peripheral end 534a and the second peripheral end 534b, scraping, deformation, or the like is unlikely to occur in the guide projection 537 and the guide groove 534, which can lead to improvement in long-term operational reliability of the valve 500.

On the other hand, when the CPAP apparatus 200 is started, as illustrated in FIG. 31, the rotary plate 530 can be positioned at a reference position P3 by utilizing the contact between the first peripheral end 534a and the guide projection 537 in the guide groove 534. For that reason, it is not necessary to detect the rotational position of the rotary plate 530 utilizing a detection sensor or the like, for example. For that reason, it is possible to suppress the number of parts, which leads to simplification of the configuration and cost reduction.

The details will be described.

In a case where the detection sensor or the like is not used, the rotational position of the rotary plate 530 cannot be grasped. Thus, after the starting of the CPAP apparatus 200, the drive motor 550 forcibly rotates the rotary plate 530 in the counterclockwise direction, and as illustrated in FIG. 31, is rotated until the first peripheral end 534a of the guide groove 534 contact the guide projection 537. Then, the origin of the rotary plate 530 is set (the origin of the pulse is set) with the position where the guide projection 537 and the first peripheral end 534a of the guide groove 534 have contacted each other as the reference position (base point) P3.

Accordingly, by performing the rotation control (the number of steps) of the rotary plate 530 with the reference position P3 as a reference, the rotary plate 530 can be appropriately positioned at the fully closed position P1 and the fully open position P2, and the rotation amount of the rotary plate 530 can be to appropriately controlled.

For that reason, after the rotary plate 530 reaches the reference position P3, the rotary plate 530 can be positioned at the fully closed position P1 illustrated in FIG. 29 by rotating the rotary plate 530 slightly in the clockwise direction in a predetermined number of steps.

Moreover, by rotating the output shaft 551 via the motor shaft in a predetermined number of steps with the fully closed position P1 as a reference, the rotary plate 530 can be rotated in the clockwise direction around the second axis 02 and can be moved toward the fully open position P2 illustrated in FIG. 28. Therefore, the rotary plate 530 can be controlled to stop at a predetermined rotational position, and the opening degree of the air discharge hole 525 through the air communication hole 533 can be linearly and accurately controlled within a range of 0% to 100%.

In addition to that, during the opening and closing operation of the valve 500, even in a case where the rotational position of the rotary plate 530 is offset due to some reason (for example, step-out of the drive motor 550), the guide projection 537 can be brought into contact with the first peripheral end 534a and the second peripheral end 534b. Therefore, it is possible to restrict any further rotation of the rotary plate 530. That is, since the movable range (rotational range) of the rotary plate 530 can be limited within the range of the guide groove 534 (that is, within the range between the first peripheral end 534a and the second peripheral end 534b), it is possible to prevent the inconvenience that the rotary plate 530 unintentionally deviates without any limitation. Therefore, the reliability of the opening and closing operation of the valve 500 can be improved.

In addition, in the above-described case, even in a case where the guide projection 537 and the first peripheral end 534a have contacted with each other, the closing region 532 can be utilized to maintain a fully closed state of the air discharge hole 525. In addition, even in a case where the guide projection 537 and the second peripheral end 534b have contacted each other, the air discharge hole 525 is maintained in a state close to a substantially fully open state, for example, the opening degree of the air discharge hole 525 is maintained at about 90 to 95%, and the opening operation of the valve 500 is unlikely to be significantly affected.

In addition, even in a case where the above-described rotational position deviation of the rotary plate 530 has occurred, the position of the rotary plate 530 can be grasped as mentioned earlier, for example, by restarting the CPAP apparatus 200, or by normally turning off the power, and then normally turning on the power when using the next day. Thus, it is possible to perform a normal operation.

In addition, in the above modification example, for example, a detection sensor (for example, a transmissive photosensor) that detects the position of the first peripheral end 534a of the guide groove 534 is provided on the side of the base plate 520, and the origin detection of the rotary plate 530 or the detection of the rotational position of the rotary plate 530 may be performed in non-contact on the basis of the reflectance of the detected light.

In particular, in a case where the detection sensor is utilized to detect the rotational position of the rotary plate 530, the above-described guide groove 534 and guide projection 537 can be omitted.

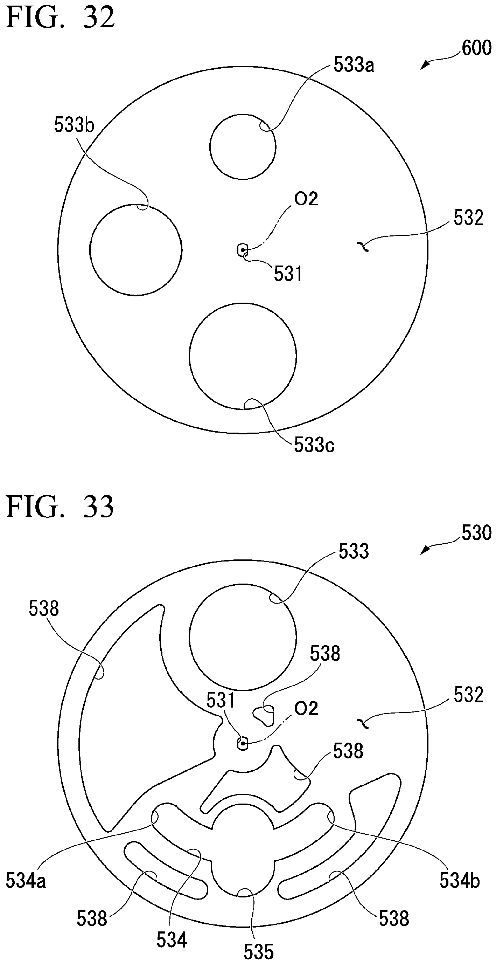

Moreover, in the above modification example, as illustrated in FIG. 32, for example, a rotary plate 600 may be adopted in which a plurality of air communication holes 533 having different opening areas are formed around the second axis 02.

In the illustrated example, the three air communication holes 533, that is, a first air communication hole 533a, a second air communication hole 533b, and a third air communication hole 533c are disposed at intervals of 90 degrees around the second axis 02. Among these holes, the first air communication hole 533a has the smallest opening area, and allows the opening degree of the air discharge hole 525 to be, for example, about 20% to 40%. The second air communication hole 533b has an opening area larger than that of the first air communication hole 533a, and allows the opening degree of the air discharge hole 525 to be, for example, about 60% to 80%. The third air communication hole 533c has the largest opening area, and allows the opening degree of the air discharge hole 525 to be, for example, 100%.

In addition, the portion of the rotary plate 600 located between the first air communication hole 533a and the third air communication hole 533c is the closing region 532 that fully closes the air discharge hole 525.

In a case where the rotary plate 600 is configured in this way, for example, the first air communication hole 533a, the second air communication hole 533b, and the third air communication hole 533c can be made to sequentially communicate with the air discharge hole 525 by rotating the rotary plate 600 every 90 degrees from the fully closed position P1 where the closing region 532 closes the air discharge hole 525. Therefore, when the rotary plate 600 is moved from the fully closed position P1 where the closing region 532 closes the air discharge hole 525 to the fully open position P2 where the third air communication hole 533c communicates with the air discharge hole 525, the opening degree of the air discharge hole 525 can be changed stepwise. Therefore, the opening degree of the valve 500 can be controlled easily and simply.

Moreover, in the above modification example, as illustrated in FIG. 33, a plurality of lightening holes 538 may be formed in a portion of the rotary plate 530 excluding the air communication hole 533, the guide groove 534 and the closing region 532.

In this case, the weight of the rotary plate 530 can be reduced by the number of the plurality of lightening holes 538, and the torque for rotating the rotary plate 530 can be suppressed. Therefore, this can lead to the power saving of the drive motor 550.

In addition, in the above modification example, the rotary plate 530 that rotates around the second axis 02 has been described as an example of the movable plate. However, the present invention is not limited to this case. For example, a movable plate that linearly reciprocates between the fully closed position P1 and the fully open position P2 may be used. However, the use of the rotary plate 530 is more preferable because the rotary plate can be moved in a space-saving manner and the valve 500 itself can be easily downsized.

Although the embodiments of the present invention have been described above, these embodiments are presented as examples and are not intended to limit the scope of the invention. The embodiments can be implemented in various other forms, and various omissions, substitutions, and changes can be made without departing from the spirit of the invention. The embodiments and the modification examples thereof include, for example, those that can be easily conceived by those skilled in the art, those that are substantially the same, and those that are in the equivalent range.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

### Industrial Applicability

According to the present invention, the CPAP system and CPAP apparatus that can improve the difficulty in breathing of the patient using CPAP can be provided. Accordingly, industrial applicability can be realized.

### Reference Signs List

R: accommodation space
P1: fully closed position
P2: fully open position
O2: second axis (rotation axis)
100, 100a: CPAP system
200, 200a: CPAP apparatus
210: fan
220: pressure sensor
220a: flow rate sensor
230: motor
240, 240a: motor drive unit
250,500: valve
260: valve drive unit
270: case
300: tube
301: exhaust hole (first air discharge hole)
400: mask
510: support plate (first fixing member)
520: base plate (second fixing member)
525: air discharge hole (second air discharge hole)
530, 600: rotary plate (movable plate)
533: air communication hole
534: guide groove
534a, 534b: peripheral end
537: guide projection

## Claims

1. A CPAP system comprising:
a fan that suctions air and delivers the air;
a sensor that measures a pressure or flow rate of the air delivered by the fan;
a case having the fan and the sensor built therein and having an air inflow port that allows the air sent into the fan to flow in therethrough and an air outflow port that allows the air delivered from the fan to flow out therethrough;
a motor drive unit that controls a rotation speed of a motor, which rotates the fan on the basis of the pressure or the flow rate measured by the sensor, by a PWM signal;
a valve that regulates a pressure or flow rate of air supplied from the air outflow port via a tube to a mask worn on a patient; and
a valve drive unit that opens and closes the valve on the basis of the PWM signal,
wherein the valve drive unit includes a synthesis unit that synthesizes three-phase signals for driving the motor, on the basis of the PWM signal, and a control unit that controls a closing speed of the valve and an opening degree of the valve, and
wherein the valve drive unit opens and closes the valve on the basis of a synthesized signal obtained by the synthesis unit synthesizing the three-phase signals, and the closing speed and the opening degree of the valve controlled by the control unit.

2. The CPAP system according to claim 1,
wherein the control unit controls an opening speed of the valve.

3. The CPAP system according to claim 2,
wherein the valve drive unit includes a determination unit that determines the rotation speed of the motor on the basis of a duty ratio of the synthesized signal, and
wherein the valve drive unit opens and closes the valve on the basis of a result obtained by the determination unit determining the rotation speed of the motor.

4. The CPAP system according to claim 3,
wherein the determination unit determines that the motor is accelerating in a case where a value obtained by converting the duty ratio of the synthesized signal into a voltage is equal to or higher than a threshold value, and determines that the motor is decelerating in a case where a value obtained by converting the duty ratio of the synthesized signal into a voltage is less than the threshold value.

5. The CPAP system according to claim 4,
wherein the control unit generates a control signal for opening and closing the valve on the basis of the opening degree and the opening and closing speed of the valve.

6. A CPAP apparatus comprising:
a fan that suctions air and delivers the air;
a sensor that measures a pressure or flow rate of the air delivered by the fan;
a case having the fan and the sensor built therein and having an air inflow port that allows the air sent into the fan to flow in therethrough and an air outflow port that allows the air delivered from the fan to flow out therethrough;
a motor drive unit that controls a rotation speed of a motor, which rotates the fan on the basis of the pressure or the flow rate measured by the sensor, by a PWM signal;
a valve that regulates a pressure or flow rate of air supplied from the air outflow port via a tube to a mask worn on a patient; and
a valve drive unit that opens and closes the valve on the basis of the PWM signal,
wherein the valve drive unit includes a synthesis unit that synthesizes three-phase signals for driving the motor, on the basis of the PWM signal, and a control unit that controls a closing speed of the valve and an opening degree of the valve, and
wherein the valve drive unit opens and closes the valve on the basis of a synthesized signal obtained by the synthesis unit synthesizing the three-phase signals, and the closing speed and the opening degree of the valve controlled by the control unit.

7. The CPAP system according to any one of claims 1 to 5,
wherein the valve includes
a first fixing member that is disposed on an outer peripheral side of the tube and combined with the tube;
a second fixing member that is combined with the first fixing member in a state where an accommodation space is formed between the first fixing member and the second fixing member; and
a movable plate that is disposed in the accommodation space and is movable relative to the first fixing member and the second fixing member,
wherein an inside of the accommodation space communicates with an inside of the tube through a first air discharge hole formed in the tube,
wherein a second air discharge hole communicating with the outside is formed in the second fixing member,
wherein the movable plate is disposed so as to allow closing of the second air discharge hole, and has at least one air communication hole that allows communication between the inside of the second air discharge hole and the inside of the accommodation space, and
wherein the movable plate is reciprocable between a fully closed position where the second air discharge hole is fully closed and a fully open position where the second air discharge hole is fully opened through the at least one air communication hole.

8. The CPAP system according to claim 7,
wherein the movable plate gradually increases an opening degree of the second air discharge hole through the air communication hole as the movable plate moves from the fully closed position to the fully open position.

9. The CPAP system according to claim 7,
wherein a plurality of the air communication holes are formed so as to be lined up along a movement direction of the movable plate, and are formed so as to have different opening areas,
wherein the movable plate is movable from the fully closed position toward the fully open position such that the plurality of air communication holes sequentially communicate with the second air discharge hole, and
wherein the second air discharge hole changes in opening degree depending on communication with each of the plurality of air communication holes, and is fully opened by communication with the air communication hole having a largest opening area when the movable plate is located at the fully open position.

10. The CPAP system according to any one of claims 7 to 9,
wherein the movable plate is reciprocally rotatable between the fully closed position and the fully open position around a rotation axis.

11. The CPAP system according to claim 10,
wherein a guide groove is formed in the movable plate so as to extend in a circumferential direction around the rotation axis, and
wherein at least one of the first fixing member and the second fixing member is formed with a guide projection that is inserted into the guide groove.

12. The CPAP apparatus according to claim 6,
wherein the valve includes
a first fixing member that is disposed on an outer peripheral side of the tube and combined with the tube;
a second fixing member that is combined with the first fixing member in a state where an accommodation space is formed between the first fixing member and the second fixing member; and
a movable plate that is disposed in the accommodation space and is movable relative to the first fixing member and the second fixing member,
wherein an inside of the accommodation space communicates with an inside of the tube through a first air discharge hole formed in the tube,
wherein a second air discharge hole communicating with the outside is formed in the second fixing member,
wherein the movable plate is disposed so as to allow closing of the second air discharge hole, and has at least one air communication hole that allows communication between the inside of the second air discharge hole and the inside of the accommodation space, and
wherein the movable plate is reciprocable between a fully closed position where the second air discharge hole is fully closed and a fully open position where the second air discharge hole is fully opened through the at least one air communication hole.
